# EUROPEAN PATENT SPECIFICATION

(11) **EP 4 119 569 B1**
(45) Date of publication and mention of the grant of the patent: **31.07.2024**
(21) Application number: 22180697.9
(22) Date of filing: 07.11.2016
(51) Int. Cl.: C07H 19/20, C07H 21/02, C07H 21/04, C12N 15/113, A61P 3/06

(54) **CONJUGATED ANTISENSE COMPOUNDS FOR USE IN THERAPY**
KONJUGIERTE ANTISENSE-VERBINDUNGEN ZUR VERWENDUNG IN DER THERAPIE
COMPOSÉS ANTISENS CONJUGUÉS À UTILISER EN THÉRAPIE

(30) Priority: 06.11.2015 US 201562252397 P
(43) Date of publication of application: 18.01.2023
(62) Divisional of application: 16863168.7
(73) Proprietor: Ionis Pharmaceuticals, Inc., Carlsbad, CA 92010 (US)
(72) Inventor: VINEY, Nicholas, J., Carlsbad, 92010 (US); GEARY, Richard, S., Carlsbad, 92010 (US); WANG, Yanfeng, Carlsbad, 92010 (US); YU, Zhengrong, Carlsbad, 92010 (US); GUNAWAN, Rudy, Carlsbad, 92010 (US)
(74) Representative: Carpmaels & Ransford LLP

(56) References cited:
- WO-A1-2017/079739
- WO-A2-2014/205451
- WO-A2-2015/168589
- US-A1- 2009 018 350
- US-A1- 2015 126 719
- VINEY NICHOLAS J ET AL: "Antisense oligonucleotides targeting apolipoprotein(a) in people with raised lipoprotein(a): two randomised, double-blind, placebo-controlled, dose-ranging trials", LANCET, ELSEVIER, AMSTERDAM, NL, vol. 388, no. 10057, 21 September 2016 (2016-09-21), pages 2239 - 2253, XP029801392, ISSN: 0140-6736, DOI: 10.1016/S0140-6736(16)31009-1

## Description

### BACKGROUND OF THE INVENTION

The principle behind antisense technology is that an antisense compound hybridizes to a target nucleic acid and modulates the amount, activity, and/or function of the target nucleic acid. For example in certain instances, antisense compounds result in altered transcription or translation of a target. Such modulation of expression can be achieved by, for example, target mRNA degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi refers to antisense-mediated gene silencing through a mechanism that utilizes the RNA-induced siliencing complex (RISC). An additional example of modulation of RNA target function is by an occupancy-based mechanism such as is employed naturally by microRNA. MicroRNAs are small non-coding RNAs that regulate the expression of protein-coding RNAs. The binding of an antisense compound to a microRNA prevents that microRNA from binding to its messenger RNA targets, and thus interferes with the function of the microRNA. MicroRNA mimics can enhance native microRNA function. Certain antisense compounds alter splicing of pre-mRNA. Regardless of the specific mechanism, sequence-specificity makes antisense compounds attractive as tools for target validation and gene functionalization, as well as therapeutics to selectively modulate the expression of genes involved in the pathogenesis of diseases.

Antisense technology is an effective means for modulating the expression of one or more specific gene products and can therefore prove to be uniquely useful in a number of therapeutic, diagnostic, and research applications. Chemically modified nucleosides may be incorporated into antisense compounds to enhance one or more properties, such as nuclease resistance, pharmacokinetics or affinity for a target nucleic acid. In 1998, the antisense compound, Vitravene^{®} (fomivirsen; developed by Isis Pharmaceuticals Inc., Carlsbad, CA) was the first antisense drug to achieve marketing clearance from the U.S. Food and Drug Administration (FDA), and is currently a treatment of cytomegalovirus (CMV)-induced retinitis in AIDS patients.

New chemical modifications have improved the potency and efficacy of antisense compounds, uncovering the potential enhancing subcutaneous administration, decreasing potential for side effects, and leading to improvements in patient convenience. Chemical modifications increasing potency of antisense compounds allow administration of lower doses, which reduces the potential for toxicity, as well as decreasing overall cost of therapy. Modifications increasing the resistance to degradation result in slower clearance from the body, allowing for less frequent dosing. Different types of chemical modifications can be combined in one compound to further optimize the compound's efficacy.

One chemical modification used to improve the activity of RNAse H dependent (gapmer) antisense compounds *in vivo* is conjugation to a conjugate group, such as a GalNAc cluster. Conjugation to a conjugate group has been shown to improve potency in vivo in non-human subjects, for example including the use of RNAse H dependent (gapmer) antisense compounds conjugated to GalNAc clusters as disclosed in WO 2014/179620. Prior to the present invention, no RNAse H dependent (gapmer) antisense compounds conjugated to GalNAc clusters had been tested in humans to achieve target reduction.

WO 2014/205451 A2 describes chemically-modified oligonucleotides for use in research, diagnostics, and/or therapeutics. Also described are compounds and methods for the modulation of a target nucleic acid and compounds and methods for the modulation of Apoliprotein C-III expression.

US2015126719 A1 describes oligomeric compounds with conjugate groups targeting apoplipoprotein C-III (ApoCIII). For example, the ApoCIII targeting oligomeric compounds are conjugated to N-Acetylgalactosamine. Also described are conjugated oligomeric compounds targeting ApoCIII for use in decreasing ApoCIII to treat, prevent, or ameliorate diseases, disorders or conditions related to ApoCIII. Certain diseases, disorders or conditions related to ApoCIII include inflammatory, cardiovascular and/or metabolic diseases, disorders or conditions. The conjugated oligomeric compounds can be used to treat such diseases, disorders or conditions in an individual in need thereof.

WO2015168589 A2 describes methods, compounds, and compositions for reducing expression of an ANGPTL3 mRNA and protein in an animal. Also described are methods, compounds, and compositions for reducing lipids and/or glucose in an animal. Such methods, compounds, and compositions are useful to treat, prevent, delay, or ameliorate any one or more of cardiovascular disease and/or metabolic disease, or a symptom thereof, in an individual in need thereof.

US 2009/018350 A1 describes a fluorine-containing ether compound with a fluorine content increased, the fluorine content being enhanced by fluorinating a polymer including a repeating unit represented by Formula (I), wherein Rh₁ represents a divalent linkage group; Rf₂ represents an perfluoroalkylene group; each of Rf₃ and Rf₄ independently represents a fluorine atom, a perfluoroalkyl group or a perfluoroalkoxy group, and any two of Rf₂, Rf₃ and Rf₄ may be combined with each other to form a ring.

### SUMMARY OF THE INVENTION

The invention is defined in the appended claims. Any disclosure going beyond the scope of said claims is only intended for illustrative purposes and is thus not part of the invention.

Furthermore, the references to methods of treatment in the subsequent paragraphs of this description are to be interpreted as references to the compounds, pharmaceutical compositions of the present invention for use in a method for treatment of the human (or animal) body by therapy (or for diagnosis).

The present invention provides an oligomeric compound for use in treating or preventing a disease or condition in a human, wherein the treatment comprises administering not more than 300mg of the oligomeric compound to the human during a dosing period of four weeks, wherein the anion form of the oligomeric compound has the following chemical structure:

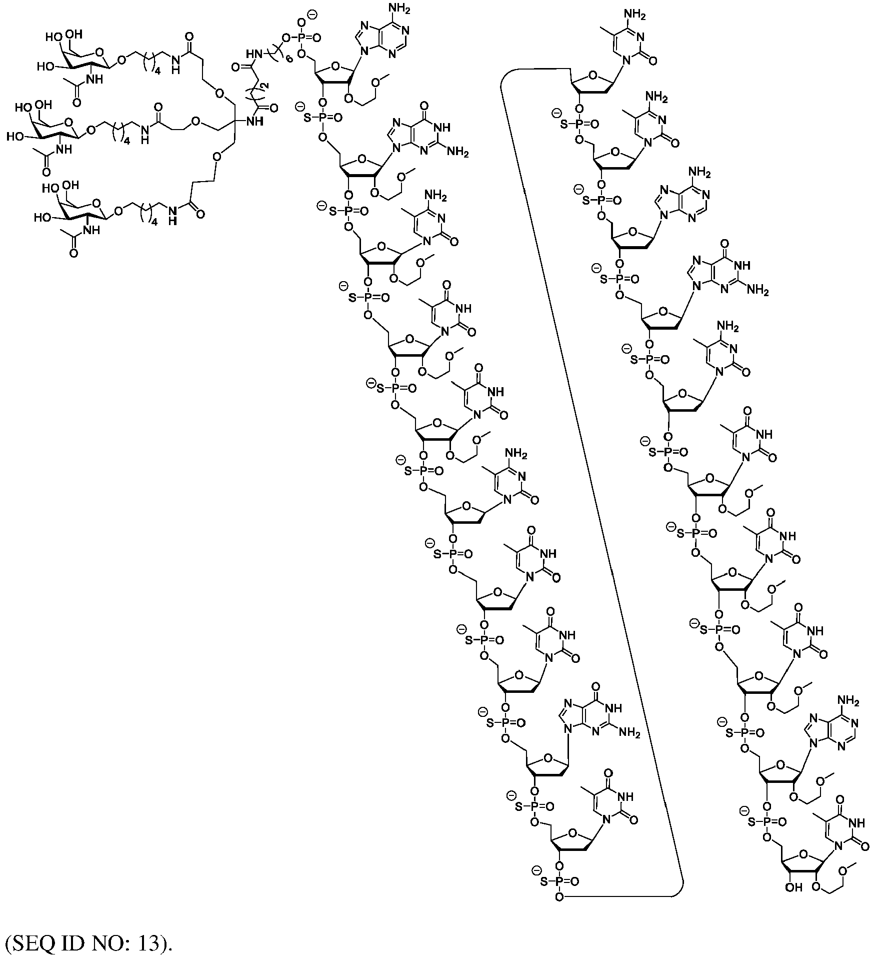

While it was known that oligomeric gapmer compounds comprising a GalNAc cluster had improved *in vivo* potency from work in non-human subjects *(e.g.* WO 2014/179620), the inventors were the first to test this class of compounds in humans. It was discovered that the oligomeric gapmer compounds comprising a GalNAc cluster are particularly effective when administered to a human subject. The improvement provided in humans was unexpectedly greater than the improvement seen in the non-human subjects. Amongst the improvements observed included increased potency relative to that expected from the earlier work using non-human subjects. A further improvement observed included increased half-life relative to that expected from the work using non-human subjects.

Following this discovery, one aspect of the invention is oligomeric gapmer compounds comprising a GalNAc cluster for use a method of treating a disease or condition in a human by using lower than expected doses, and yet still providing excellent reduction of a given target nucleic acid. In addition, following the first testing of these oligomeric gapmer compounds in humans, a further aspect of the invention is that the oligomeric gapmer compounds comprising a GalNAc cluster may be administered to a human subject only once a week, only once a month, or only once every three months, and yet still provide excellent reduction of a given target nucleic acid. *See,* e.g., Viney, et al. Lancet, 2016, Sep 2016; 388: 2239-53.

The invention also provides unit dosage forms with low amounts of the oligomeric gapmer compound useful in these methods thanks to their relatively low drug amounts.

The present invention provides the following numbered embodiments:
Embodiment 1: An oligomeric compound for use in treating or preventing a disease or condition in a human, wherein the treatment comprises administering not more than 300mg of the oligomeric compound to the human during a dosing period of four weeks, wherein the anion form of the oligomeric compound has the following chemical structure: 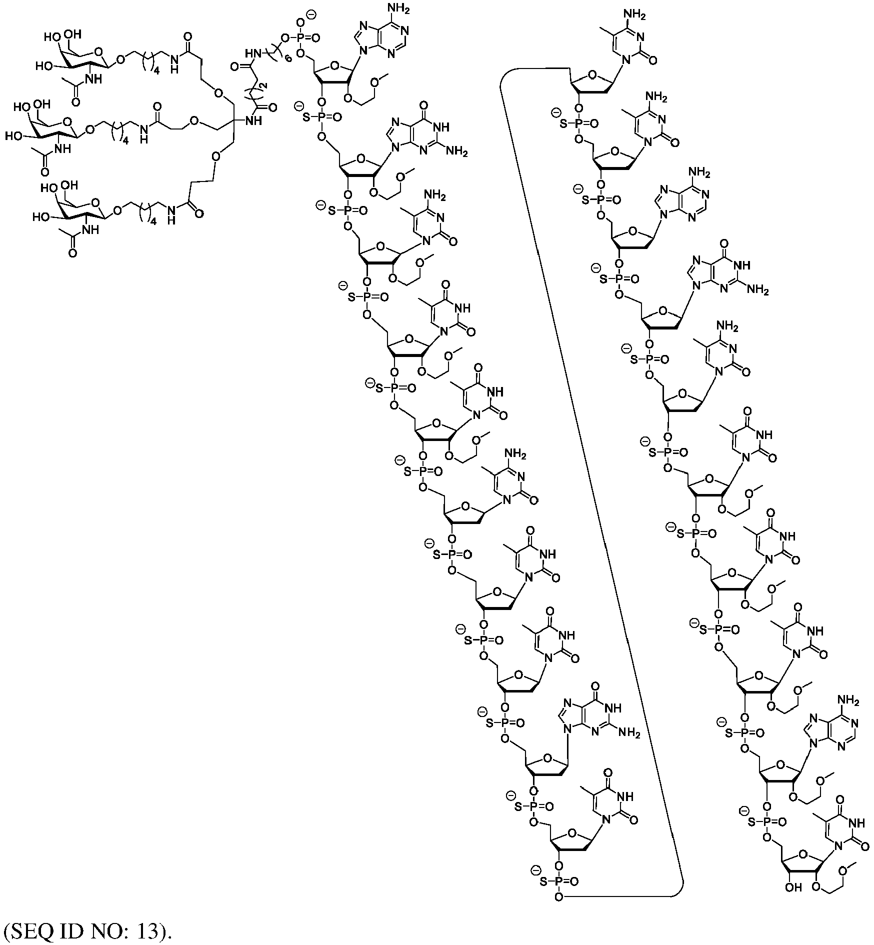
Embodiment 2: The oligomeric compound for use according to embodiment 1, wherein the treatment comprises administering not more than 250mg of the oligomeric compound to the human during the dosing period.
Embodiment 3: The oligomeric compound for use according to any preceding embodiment, wherein the treatment comprises administering not more than 100mg of the oligomeric compound to the human during the dosing period.
Embodiment 4: The oligomeric compound for use according to any preceding embodiment, wherein the treatment comprises administering a unit dose comprising not more than 250mg of the oligomeric compound.
Embodiment 5: The oligomeric compound for use according to any preceding embodiment, wherein the treatment comprises administering a unit dose comprising not more than 100mg of the oligomeric compound.
Embodiment 6: The oligomeric compound for use according to any preceding embodiment, wherein the treatment comprises administering a unit dose comprising not more than 75mg of the oligomeric compound.
Embodiment 7: The oligomeric compound for use according to any preceding embodiment, wherein the treatment comprises administering a unit dose comprising not more than 50mg of the oligomeric compound.
Embodiment 8: The oligomeric compound for use according to any preceding embodiment, wherein the treatment comprises administering a unit dose comprising not more than 40mg of the oligomeric compound.
Embodiment 9: The oligomeric compound for use according to any preceding embodiment, wherein the treatment comprises administering a unit dose comprising not more than 30mg of the oligomeric compound.
Embodiment 10: The oligomeric compound for use according to any preceding embodiment, wherein the treatment comprises administering a unit dose comprising not more than 25mg of the oligomeric compound.
Embodiment 11: The oligomeric compound for use according to any preceding embodiment, wherein the treatment comprises administering a unit dose comprising not more than 20mg of the oligomeric compound.
Embodiment 12: The oligomeric compound for use according to any preceding embodiment, wherein the treatment comprises administering a unit dose comprising not more than 15mg of the oligomeric compound.
Embodiment 13: The oligomeric compound for use according to any of embodiments 1 to 3, wherein the treatment comprises administering a unit dose of from 75mg to 85mg, optionally 80 mg.
Embodiment 14: The oligomeric compound for use according to any of embodiments 1 to 3, wherein the treatment comprises administering a unit dose of from 55mg to 65mg, optionally 60 mg.
Embodiment 15: The oligomeric compound for use according to any of embodiments 1 to 3, wherein the treatment comprises administering a unit dose of from 45mg to 55mg, optionally 50 mg.
Embodiment 16: The oligomeric compound for use according to any of embodiments 1 to 3, wherein the treatment comprises administering a unit dose of from 35mg to 45mg, optionally 40 mg.
Embodiment 17: The oligomeric compound for use according to any of embodiments 1 to 3, wherein the treatment comprises administering a unit dose of from 25mg to 35mg, optionally 30 mg.
Embodiment 18: The oligomeric compound for use according to any of embodiments 1 to 3, wherein the treatment comprises administering a unit dose of from 15mg to 25mg, optionally 20 mg.
Embodiment 19: The oligomeric compound for use according to any of embodiments 1 to 3, wherein the treatment comprises administering a unit dose of from 5mg to 15mg, optionally 10 mg.
Embodiment 20: The oligomeric compound for use according to any of embodiments 1 to 3, wherein the treatment comprises administering a unit dose comprising not less than 1mg of the oligomeric compound.
Embodiment 21: The oligomeric compound for use according to embodiment 1 to 3, wherein the treatment comprises administering a unit dose comprising not less than 2.5mg of the oligomeric compound
Embodiment 22: The oligomeric compound for use according to embodiment 1 to 3, wherein the treatment comprises administering a unit dose comprising not less than 5mg of the oligomeric compound
Embodiment 23: The oligomeric compound for use according to any of embodiments 4-19, wherein the treatment comprises administering not more than 1 unit dose to the human during the dosing period.
Embodiment 24: The oligomeric compound for use according to any of embodiments 4-19, wherein the treatment comprises administering not more than 2 unit doses to the human during the dosing period.
Embodiment 25: The oligomeric compound for use according to any of embodiments 4-19, wherein the treatment comprises administering not more than 3 unit doses to the human during the dosing period.
Embodiment 26: The oligomeric compound for use according to any of embodiments 4-19, wherein the treatment comprises administering not more than 4 unit doses to the human during the dosing period.
Embodiment 27: The oligomeric compound for use according to any of embodiments 4-19, wherein the treatment comprises administering not more than 5 unit doses to the human during the dosing period.
Embodiment 28: The oligomeric compound for use according to any of embodiments 4-19, wherein the treatment comprises administering not more than 6 unit doses to the human during the dosing period.
Embodiment 29: The oligomeric compound for use according to any of embodiments 4-19, wherein the treatment comprises administering a loading dose.
Embodiment 30: The oligomeric compound for use according to any of embodiments 4-19, wherein the treatment comprises administering a maintenance dose.
Embodiment 31: The oligomeric compound for use according to embodiment 29 or 30, wherein the loading dose is given prior to the maintenance dose.
Embodiment 32: The oligomeric compound for use according to any of embodiments 29-31, wherein the loading dose consists of 3 unit doses administered in the loading dose period.
Embodiment 33: The oligomeric compound for use according to any of embodiments 29-31, wherein the loading dose consists of 4 unit doses administered in the loading dose period.
Embodiment 34: The oligomeric compound for use according to any of embodiments 29-31, wherein the loading dose consists of 5 unit doses administered in the loading dose period.
Embodiment 35: The oligomeric compound for use according to any of embodiments 29-31, wherein the loading dose consists of 6 unit doses administered in the loading dose period.
Embodiment 36: The oligomeric compound for use according to any of embodiments 29-35, wherein the loading dose is given over a period of 4 weeks.
Embodiment 37: The oligomeric compound for use according to embodiment 35 or 36, wherein the initial loading dose is given at day 1, and subsequent loading doses are given at days 3, 5, 8, 15, and 22.
Embodiment 38: The oligomeric compound for use according to any of embodiments 29-37, wherein the maintenance dose is given once every week.
Embodiment 39: The oligomeric compound for use according to any of embodiments 29-37, wherein the maintenance dose is given every two weeks.
Embodiment 40: The oligomeric compound for use according to any of embodiments 29-37, wherein the maintenance dose is given three weeks.
Embodiment 41: The oligomeric compound for use according to any of embodiments 29-37, wherein the maintenance dose is given every four weeks.
Embodiment 42: The oligomeric compound for use according to any of embodiments 29-37, wherein the maintenance dose is given every month.
Embodiment 43: The oligomeric compound for use according to any of embodiments 29-37, wherein the maintenance dose is given every two months.
Embodiment 44: The oligomeric compound for use according to any of embodiments 29-37, wherein the maintenance dose is given every three months.
Embodiment 45: The oligomeric compound for use according to any preceding embodiment, wherein the oligomeric compound is administered by injection.
Embodiment 46: The oligomeric compound for use according to embodiment 45, wherein the oligomeric compound is administered by subcutaneous injection, optionally by subcutaneous injection into the abdomen, thigh, or upper arm.
Embodiment 47: The oligomeric compound for use according to embodiment 45 or embodiment 46, wherein the oligomeric compound is formulated in a sterile liquid and optionally wherein each unit dose of the oligomeric compound is not more than 1 mL of the sterile liquid.
Embodiment 48: The oligomeric compound for use according to embodiment 47, wherein each unit dose of the oligomeric compound is not more than 0.8 mL of the sterile liquid.
Embodiment 49: The oligomeric compound for use according to embodiment 47, wherein each unit dose of the oligomeric compound is not more than 0.5 mL of the sterile liquid.
Embodiment 50: The oligomeric compound for use according to embodiment 47, wherein each unit dose of the oligomeric compound is not more than 0.25 mL of the sterile liquid.
Embodiment 51: The oligomeric compound for use according to any of embodiments 47 to 50, wherein the sterile liquid is selected from among: sterile saline and water.
Embodiment 52: The oligomeric compound for use according to embodiment 51, wherein the sterile liquid further comprises a buffer.
Embodiment 53: The oligomeric compound for use according to embodiment 51 or 52, wherein the sterile liquid further comprises sodium chloride.
Embodiment 54: The oligomeric compound for use according to any preceding embodiment, wherein the oligomeric compound is formulated as a sodium salt.
Embodiment 55: The oligomeric compound for use according to any preceding embodiment, wherein the oligomeric compound is targeted to a nucleic acid molecule encoding human Apolipoprotein CIII (ApoCIII).
Embodiment 56: The oligomeric compound for use according to embodiment 55, wherein the treatment reduces the fasting plasma triglyceride concentration in the human by at least 30%, when the fasting plasma triglyceride concentration in the human is measured at the start and end of the dosing period.
Embodiment 57: A pharmaceutical composition, comprising:
   (i) an oligomeric compound wherein the oligomeric compound is an oligomeric compound as defined in any one of embodiments 1-56, and
   (ii) one or more pharmaceutically acceptable carriers or diluents,
   wherein the pharmaceutical composition is formulated for use in a treatment as set forth in any one of embodiments 1-56.
Embodiment 58: A sterile sealed container which contains the pharmaceutical composition of embodiment 57.
Embodiment 59: A sterile container according to any of embodiment 57 or 58, wherein the container is a vial.
Embodiment 60: The sterile container according to any of embodiment 57 or 58, wherein the container is a syringe.
Embodiment 61: The sterile container according to embodiment 59 or 60, wherein the container is for single use.
Embodiment 62: A packaged pharmaceutical product comprising: (a) multiple unit dosage forms each comprising a sealed sterile container according to any of embodiments 58-60; and (b) printed instructions describing the administration of the unit dosage forms for a treatment as set forth in any of embodiments 1-56.
Embodiment 63: An oligomeric compound for use in a method of treating or preventing a disease or condition in a human, wherein the method comprises administering a unit dose of the oligomeric compound to the human subject in need thereof, and not more than 300mg of the oligomeric compound to the human during a dosing period of 4 weeks, wherein the anion form of the oligomeric compound has the following chemical structure: 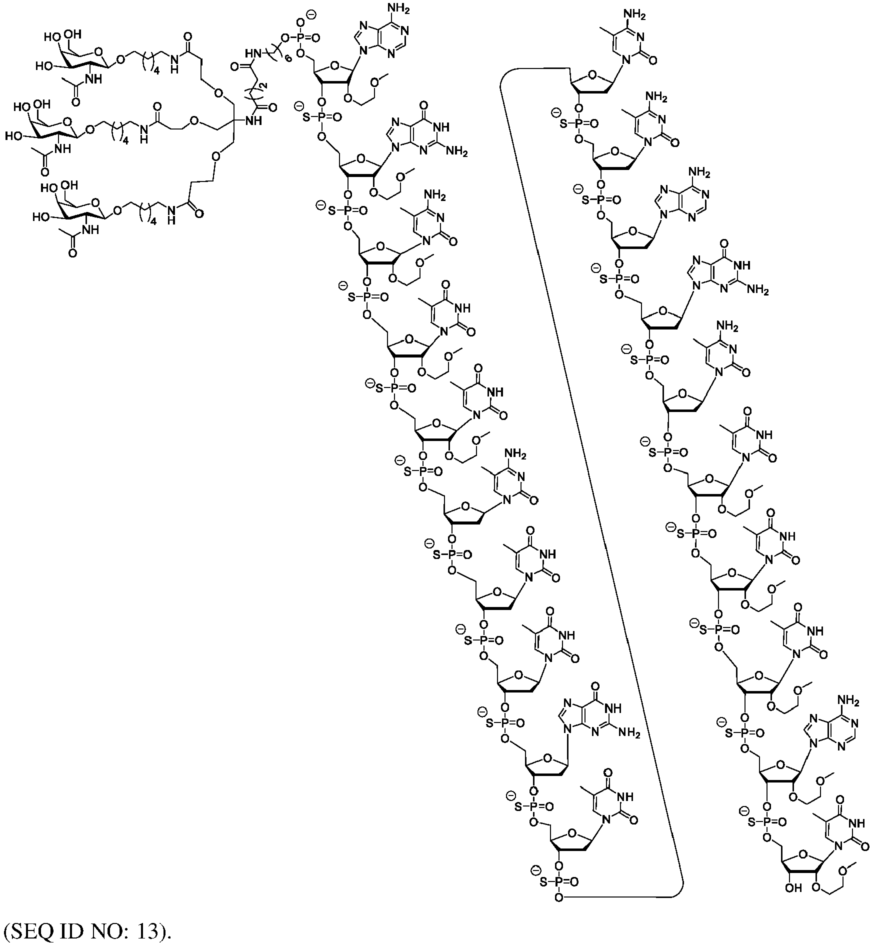
Embodiment 64: The oligomeric compound for use according to embodiment 63, wherein the unit dose is 120 mg.
Embodiment 65: The oligomeric compound for use according to embodiment 63, wherein the unit dose is 100 mg.
Embodiment 66: The oligomeric compound for use according to embodiment 63, wherein the unit dose is 80 mg.
Embodiment 67: The oligomeric compound for use according to embodiment 63, wherein the unit dose is 60 mg.
Embodiment 68: The oligomeric compound for use according to embodiment 63, wherein the unit dose is 40 mg.
Embodiment 69: The oligomeric compound for use according to embodiment 63, wherein the unit dose is 30 mg.
Embodiment 70: The oligomeric compound for use according to embodiment 63, wherein the unit dose is 20 mg.
Embodiment 71: The oligomeric compound for use according to embodiment 63, wherein the unit dose is 15 mg.
Embodiment 72: The oligomeric compound for use according to embodiment 63, wherein the unit dose is 10 mg.
Embodiment 73: The oligomeric compound for use according to any of embodiments 63 to 72, wherein the unit dose is administered once every week.
Embodiment 74: The oligomeric compound for use according to any of embodiments 63 to 72, wherein the unit dose is administered once every 2 weeks.
Embodiment 75: The oligomeric compound for use according to any of embodiments 63 to 72, wherein the unit dose is administered once every 3 weeks.
Embodiment 76: The oligomeric compound for use according to any of embodiments 63 to 72, wherein the unit dose is administered once every 4 weeks.
Embodiment 77: The oligomeric compound for use according to any of embodiments 63 to 72, wherein the unit dose is administered once every month.
Embodiment 78: The oligomeric compound for use according to any of embodiments 63 to 72, wherein the unit dose is administered once every 2 months.
Embodiment 79: The oligomeric compound for use according to any of embodiments 63 to 72, wherein the unit dose is administered once every 3 months.
Embodiment 80: The oligomeric compound for use according to any of embodiments 63 to 72, wherein the unit dose is administered on day 1, 3, 5, 8, 15, 22, and once per week thereafter.
Embodiment 81: The oligomeric compound for use according to any of embodiments 63 to 80, wherein the subject has one or more symptoms of a cardiovascular disease or disorder.
Embodiment 82: The oligomeric compound for use according to embodiment 81, wherein one or more symptoms of the cardiovascular disease or disorder are ameliorated.

In certain embodiments, the present invention provides an oligomeric compound for use in treating or preventing a disease or condition in a human, wherein the treatment comprises administering one or more doses of the oligomeric compound to the human in (a) a loading or induction phase, and (b) a maintenance phase. In certain embodiments, a dose of the oligomeric compound is administered to the human during the maintenance phase once per week, once every two weeks, once per month, once every two months or once quarterly, for as long as needed, effective, and/or tolerated.

The treatment comprises administering not more than not more than 300mg of the oligomeric compound to the human during the dosing period of four weeks.

### BRIEF DESCRIPTION OF THE FIGURES

Figures 1A-C illustrate the predicted Lp(a) levels as a result of different weekly dosing regimens. Doses of 20 mg (Fig 1A), 30 mg (Fig 1B) or 40 mg (Fig 1C) shows a steady state reduction of Lp (a) of ≥ 80%.
Figures 2A-B illustrate the predicted Lp(a) levels as a result of different monthly dosing regimens. Doses of 60 mg (Fig 2A) and 80 mg (Fig 2B) Lp(a) show a steady state reduction of Lp (a) of about 80%.
Figure 3 illustrates the predicted Lp (a) levels as a result of a 2-month dosing regimen (e.g. one dose every two months). An 80 mg dose every 2-months shows a steady state reduction of Lp (a) of about 80%.
Figure 4 illustrates the predicted Lp (a) levels as a result of a quarterly dosing regimen. An 80 mg dose every quarter shows a steady state reduction of Lp (a) of 80% and maximum reduction of Lp (a) of > 90%.
Figures 5A-D illustrate the predicted Lp(a) levels as a result of different monthly dosing regimens. Figures are shown modeling the effect on Lp(a) by monthly administration of ISIS 681257 at doses of 20 mg (Fig 5A), 40 mg (Fig. 5B), 60 mg (Fig. 5C), and 80 mg (Fig.5D ). The dark middle line represents the predicted dose, while the uppermost and lowermost lines represent the 90% Confidence Interval.
Figures 6A-D illustrate the predicted Lp(a) levels as a result of different weekly dosing regimens. Figures 6A-D show modeling of the effect on Lp(a) by weekly administration of ISIS 681257 at doses of 5 mg (Fig 6A), 10 mg (Fig. 6B), 20 mg (Fig. 6C), and 30 mg (Fig. 6D). The dark middle line represents the predicted dose, while the uppermost and lowermost lines represent the 90% Confidence Interval.

### DETAILED DESCRIPTION

It is to be understood that both the foregoing general description and the following detailed description are exemplary and explanatory only and are not restrictive of the disclosure. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### A. Definitions

Unless specific definitions are provided, the nomenclature used in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society, Washington D.C., 1994; "Remington's Pharmaceutical Sciences," Mack Publishing Co., Easton, Pa., 21st edition, 2005; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989.

Unless otherwise indicated, the following terms have the following meanings:
As used herein, "2'-deoxynucleoside" means a nucleoside comprising 2'-H(H) furanosyl sugar moiety, as found in naturally occurring deoxyribonucleic acids (DNA). In certain embodiments, a 2'-deoxynucleoside may comprise a modified nucleobase or may comprise an RNA nucleobase (uracil).

As used herein, "2'-substituted nucleoside" or "2-modified nucleoside" means a nucleoside comprising a 2'-substituted or 2'-modified sugar moiety. As used herein, "2'-substituted" or "2-modified" in reference to a sugar moiety means a sugar moiety comprising at least one 2'-substituent group other than H or OH.

As used herein, "antisense activity" means any detectable and/or measurable change attributable to the hybridization of an antisense compound to its target nucleic acid. In certain embodiments, antisense activity is a decrease in the amount or expression of a target nucleic acid or protein encoded by such target nucleic acid compared to target nucleic acid levels or target protein levels in the absence of the antisense compound.

As used herein, "antisense compound" means a compound comprising an antisense oligonucleotide and optionally one or more additional features, such as a conjugate group or terminal group.

As used herein, "antisense oligonucleotide" means an oligonucleotide having a nucleobase sequence that is at least partially complementary to a target nucleic acid.

As used herein, "ameliorate" in reference to a treatment means improvement in at least one symptom relative to the same symptom in the absence of the treatment. In certain embodiments, amelioration is the reduction in the severity or frequency of a symptom or the delayed onset or slowing of progression in the severity or frequency of a symptom.

As used herein, "bicyclic nucleoside" or "BNA" means a nucleoside comprising a bicyclic sugar moiety. As used herein, "bicyclic sugar" or "bicyclic sugar moiety" means a modified sugar moiety comprising two rings, wherein the second ring is formed via a bridge connecting two of the atoms in the first ring thereby forming a bicyclic structure. In certain embodiments, the first ring of the bicyclic sugar moiety is a furanosyl moiety. In certain embodiments, the bicyclic sugar moiety does not comprise a furanosyl moiety.

As used herein, "branching group" means a group of atoms having at least 3 positions that are capable of forming covalent linkages to at least 3 groups. In certain embodiments, a branching group provides a plurality of reactive sites for connecting tethered ligands to an oligonucleotide via a conjugate linker and/or a cleavable moiety.

As used herein, "cell-targeting moiety" means a conjugate group or portion of a conjugate group that is capable of binding to a particular cell type or particular cell types.

As used herein, "cleavable moiety" means a bond or group of atoms that is cleaved under physiological conditions, for example, inside a cell, an animal, or a human.

As used herein, "complementary" in reference to an oligonucleotide means that at least 70% of the nucleobases of such oligonucleotide or one or more regions thereof and the nucleobases of another nucleic acid or one or more regions thereof are capable of hydrogen bonding with one another when the nucleobase sequence of the oligonucleotide and the other nucleic acid are aligned in opposing directions. Complementary nucleobases means nucleobases that are capable of forming hydrogen bonds with one another. Complementary nucleobase pairs include adenine (A) and thymine (T), adenine (A) and uracil (U), cytosine (C) and guanine (G), 5-methyl cytosine (^{m}C) and guanine (G). Complementary oligonucleotides and/or nucleic acids need not have nucleobase complementarity at each nucleoside. Rather, some mismatches are tolerated. As used herein, "fully complementary" or "100% complementary" in reference to oligonucleotides means that such oligonucleotides are complementary to another oligonucleotide or nucleic acid at each nucleoside of the oligonucleotide.

As used herein, "conjugate group" means a group of atoms that is directly or indirectly attached to an oligonucleotide. Conjugate groups include a conjugate moiety and a conjugate linker that attaches the conjugate moiety to the oligonucleotide.

As used herein, "conjugate linker" means a group of atoms comprising at least one bond that connects a conjugate moiety to an oligonucleotide.

As used herein, "conjugate moiety" means a group of atoms that is attached to an oligonucleotide via a conjugate linker.

As used herein, "contiguous" in the context of an oligonucleotide refers to nucleosides, nucleobases, sugar moieties, or internucleoside linkages that are immediately adjacent to each other. For example, "contiguous nucleobases" means nucleobases that are immediately adjacent to each other in a sequence.

As used herein, "double-stranded antisense compound" means an antisense compound comprising two oligomeric compounds that are complementary to each other and form a duplex, and wherein one of the two said oligomeric compounds comprises an antisense oligonucleotide.

As used herein, "fully modified" in reference to a modified oligonucleotide means a modified oligonucleotide in which each sugar moiety is modified. "Uniformly modified" in reference to a modified oligonucleotide means a fully modified oligonucleotide in which each sugar moiety is the same. For example, the nucleosides of a uniformly modified oligonucleotide can each have a 2'-MOE modification but different nucleobase modifications, and the internucleoside linkages may be different.

As used herein, "gapmer" means an antisense oligonucleotide comprising an internal region having a plurality of nucleosides that support RNase H cleavage positioned between external regions having one or more nucleosides, wherein the nucleosides comprising the internal region are chemically distinct from the nucleoside or nucleosides comprising the external regions. The internal region may be referred to as the "gap" and the external regions may be referred to as the "wings."

As used herein, "hybridization" means the pairing or annealing of complementary oligonucleotides and/or nucleic acids. While not limited to a particular mechanism, the most common mechanism of hybridization involves hydrogen bonding, which may be Watson-Crick, Hoogsteen or reversed Hoogsteen hydrogen bonding, between complementary nucleobases.

As used herein, "inhibiting the expression or activity" refers to a reduction or blockade of the expression or activity relative to the expression of activity in an untreated or control sample and does not necessarily indicate a total elimination of expression or activity.

As used herein, the terms "internucleoside linkage" means a group or bond that forms a covalent linkage between adjacent nucleosides in an oligonucleotide. As used herein "modified internucleoside linkage" means any internucleoside linkage other than a naturally occurring, phosphate internucleoside linkage. Non-phosphate linkages are referred to herein as modified internucleoside linkages. "Phosphorothioate linkage" means a modified phosphate linkage in which one of the non-bridging oxygen atoms is replaced with a sulfur atom. A phosphorothioate internucleoside linkage is a modified internucleoside linkage. Modified internucleoside linkages include linkages that comprise abasic nucleosides. As used herein, "abasic nucleoside" means a sugar moiety in an oligonucleotide or oligomeric compound that is not directly connected to a nucleobase. In certain embodiments, an abasic nucleoside is adjacent to one or two nucleosides in an oligonucleotide.

As used herein, "linker-nucleoside" means a nucleoside that links, either directly or indirectly, an oligonucleotide to a conjugate moiety. Linker-nucleosides are located within the conjugate linker of an oligomeric compound. Linker-nucleosides are not considered part of the oligonucleotide portion of an oligomeric compound even if they are contiguous with the oligonucleotide.

As used herein, "non-bicyclic modified sugar" or "non-bicyclic modified sugar moiety" means a modified sugar moiety that comprises a modification, such as a substitutent, that does not form a bridge between two atoms of the sugar to form a second ring.

As used herein, "linked nucleosides" are nucleosides that are connected in a continuous sequence (*i.e.* no additional nucleosides are present between those that are linked).

As used herein, "mismatch" or "non-complementary" means a nucleobase of a first oligonucleotide that is not complementary with the corresponding nucleobase of a second oligonucleotide or target nucleic acid when the first and second oligomeric compound are aligned.

As used herein, "MOE" means methoxyethyl. "2'-MOE" means a -OCH₂CH₂OCH₃ group at the 2' position of a furanosyl ring.

As used herein, "motif' means the pattern of unmodified and/or modified sugar moieties, nucleobases, and/or internucleoside linkages, in an oligonucleotide.

As used herein, "naturally occurring" means found in nature.

As used herein, "nucleobase" means a naturally occurring nucleobase or a modified nucleobase. As used herein a "naturally occurring nucleobase" is adenine (A), thymine (T), cytosine (C), uracil (U), and guanine (G). As used herein, a modified nucleobase is a group of atoms capable of pairing with at least one naturally occurring nucleobase. A universal base is a nucleobase that can pair with any one of the five unmodified nucleobases. As used herein, "nucleobase sequence" means the order of contiguous nucleobases in a nucleic acid or oligonucleotide independent of any sugar or internucleoside linkage modification.

As used herein, "nucleoside" means a compound comprising a nucleobase and a sugar moiety. The nucleobase and sugar moiety are each, independently, unmodified or modified. As used herein, "modified nucleoside" means a nucleoside comprising a modified nucleobase and/or a modified sugar moiety.

As used herein, "oligomeric compound" means a compound consisting of an oligonucleotide and optionally one or more additional features, such as a conjugate group or terminal group.

As used herein, "oligonucleotide" means a strand of linked nucleosides connected via internucleoside linkages, wherein each nucleoside and internucleoside linkage may be modified or unmodified. Unless otherwise indicated, oligonucleotides consist of 8-50 linked nucleosides. As used herein, "modified oligonucleotide" means an oligonucleotide, wherein at least one nucleoside or internucleoside linkage is modified. As used herein, "unmodified oligonucleotide" means an oligonucleotide that does not comprise any nucleoside modifications or internucleoside modifications.

As used herein, "pharmaceutically acceptable carrier or diluent" means any substance suitable for use in administering to an animal. Certain such carriers enable pharmaceutical compositions to be formulated as, for example, tablets, pills, dragees, capsules, liquids, gels, syrups, slurries, suspension and lozenges for the oral ingestion by a subject. In certain embodiments, a pharmaceutically acceptable carrier or diluent is sterile water; sterile saline; or sterile buffer solution.

As used herein "pharmaceutically acceptable salts" means physiologically and pharmaceutically acceptable salts of compounds, such as oligomeric compounds, *i.e.,* salts that retain the desired biological activity of the parent compound and do not impart undesired toxicological effects thereto.
As used herein "pharmaceutical composition" means a mixture of substances suitable for administering to a subject. For example, a pharmaceutical composition may comprise an antisense compound and a sterile aqueous solution. In certain embodiments, a pharmaceutical composition shows activity in free uptake assay in certain cell lines.

As used herein, "phosphorus moiety" means a group of atoms comprising a phosphorus atom. In certain embodiments, a phosphorus moiety comprises a mono-, di-, or tri-phosphate, or phosphorothioate.

As used herein "prodrug" means a therapeutic agent in a form outside the body that is converted to a differentform within the body or cells thereof, and wherein the converted form is the active form. Typically conversion of a prodrug within the body is facilitated by the action of an enzymes (e.g., endogenous or viral enzyme) or chemicals present in cells or tissues and/or by physiologic conditions.

As used herein, "RNAi compound" means an antisense compound that acts, at least in part, through RISC or Ago2 to modulate a target nucleic acid and/or protein encoded by a target nucleic acid. RNAi compounds include, but are not limited to double-stranded siRNA, single-stranded RNA (ssRNA), and microRNA, including microRNA mimics. In certain embodiments, an RNAi compound modulates the amount, activity, and/or splicing of a target nucleic acid. The term RNAi compound excludes antisense oligonucleotides that act through RNase H.

As used herein, the term "single-stranded" in reference to an antisense compound means such a compound consisting of one oligomeric compound that is not paired with a second oligomeric compound to form a duplex. "Self-complementary" in reference to an oligonucleotide means an oligonucleotide that at least partially hybridizes to itself. A compound consisting of one oligomeric compound, wherein the oligonucleotide of the oligomeric compound is self-complementary, is a single-stranded compound. A single-stranded antisense or oligomeric compound may be capable of binding to a complementary oligomeric compound to form a duplex.

As used herein, "standard cell assay" means the assay described in Example X and reasonable variations thereof.

As used herein, "standard in vivo experiment" means the procedure described in Example X and reasonable variations thereof.

As used herein, "sugar moiety" means an unmodified sugar moiety or a modified sugar moiety. As used herein, "unmodified sugar moiety" means a 2'-OH(H) furanosyl moiety, as found in RNA (an "unmodified RNA sugar moiety"), or a 2'-H(H) moiety, as found in DNA (an "unmodified DNA sugar moiety"). Unmodified sugar moieties have one hydrogen at each of the 1', 3', and 4' positions, an oxygen at the 3' position, and two hydrogens at the 5' position. As used herein, "modified sugar moiety" or "modified sugar" means a modified furanosyl sugar moiety or a sugar surrogate. As used herein, modified furanosyl sugar moiety means a furanosyl sugar comprising a non-hydrogen substituent in place of at least one hydrogen of an unmodified sugar moiety. In certain embodiments, a modified furanosyl sugar moiety is a 2'-substituted sugar moiety. Such modified furanosyl sugar moieties include bicyclic sugars and non-bicyclic sugars. As used herein, "sugar surrogate" means a modified sugar moiety having other than a furanosyl moiety that can link a nucleobase to another group, such as an internucleoside linkage, conjugate group, or terminal group in an oligonucleotide. Modified nucleosides comprising sugar surrogates can be incorporated into one or more positions within an oligonucleotide and such oligonucleotides are capable of hybridizing to complementary oligomeric compounds or nucleic acids.

As used herein, "target nucleic acid," "target RNA," "target RNA transcript" and "nucleic acid target" mean a nucleic acid that an antisense compound is designed to affect.

As used herein, "target region" means a portion of a target nucleic acid to which an antisense compound is designed to hybridize.

As used herein, "terminal group" means a chemical group or group of atoms that is covalently linked to a terminus of an oligonucleotide.

As used herein, "loading dose" means one or more doses given during the loading dose period.

As used herein, "loading dose period" means a period of time prior to the start of the maintenance dose period when one or more doses are administered to a human at a more frequent interval than during the maintenance dose period. For example, in certain embodiments, patients may receive up to 6 doses in an initial 4 week period of time, and then a subsequent maintenance dose each week after receiving the last loading dose. E.g. a patient may receive an initial dose on day 1, and subsequent doses on days 3, 5, 8, 15, and 22; and then doses every 7 days after day 22. Thus, the first six doses represent the loading dose period, and each subsequent dose administered at 7 day intervals represents the maintenance dose.

In some embodiments, the oligomeric compound is administered to the human during a loading dose period and a maintenance dose period, wherein: (i) the loading dose period precedes the maintenance dose period, (ii) the loading dose period comprises administering multiple loading doses; (iii) the maintenance dose period comprises administering multiple maintenance doses; (iv) each dose administered during the loading dose period comprises the same (mg) amount of the oligomeric compound as each dose administered during the maintenance dose period; and (v) the doses are administered less frequently during the maintenance dose period than during the loading dose period.

The loading dose period may be at least three weeks, at least four weeks, at least five weeks, at least six weeks, at least seven weeks or at least eight weeks, or the loading dose period may be at least one month, at least two months, at least three months, at least four months, at least five months or at least six months. Alternatively, the loading dose period may be up to three weeks, up to four weeks, up to five weeks, up to six weeks, up to seven weeks or up to eight weeks, or the loading dose period may be up to one month, up to two months, up to three months, up to four months, up to five months or up to six months.

The maintenance dose period may be at least three weeks, at least four weeks, at least five weeks, at least six weeks, at least seven weeks or at least eight weeks, or the maintenance dose period may be at least one month, at least two months, at least three months, at least four months, at least five months or at least six months.

As used herein, "dosing period" means the period of time between when a human subject receives the first dose and when the human subject receives a final dose. It is envisaged that dosing of the patient may continue after the end of the dosing period, such that a first dosing period is followed by one or more further dosing periods during which the same of a different dosing regimen is used. For example, a human subject may receive 6 doses in a first dosing period where the first and last dose are given 4 weeks apart. Subsequently, the human subject may then start a second dosing period where the human subject receives doses at regular intervals (e.g. one unit dose per week, one unit dose per month, or one unit dose per quarter).

As used herein, the term "unit dose" refers to the specific amount of the oligomeric compound administered to the human at a particular time point (*e.g.* the specific amount of the oligomeric compound administered to the human in a single subcutaneous injection). Each unit dose forms part of a multi-dose regimen, as described herein.

As used herein, the term "unit dosage form" denotes the physical form in which each unit dose is presented for administration.

As used herein, the term "sterile liquid" means and liquid suitable for administration to a human subject. In certain embodiments, sterile liquids comprise liquids that are substantially free from viable microorganisms or bacteria. In certain embodiments, sterile liquids comprise USP grade water or USP grade saline.

As used herein, the term "GalNac cluster" means a cell-targeting moiety having 1-4 GalNAc ligands.

### I. Certain Oligonucleotides

Oligonucleotides may be unmodified oligonucleotides (RNA or DNA) or may be modified oligonucleotides. Modified oligonucleotides comprise at least one modification relative to unmodified RNA or DNA (i.e., comprise at least one modified nucleoside (comprising a modified sugar moiety and/or a modified nucleobase) and/or at least one modified internucleoside linkage).

### A. Certain Modified Nucleosides

Modified nucleosides comprise a modified sugar moiety or a modified nucleobase or both a modifed sugar moiety and a modified nucleobase.

### 1. Certain Sugar Moieties

Examples of 2'-substituent groups suitable for non-bicyclic modified sugar moieties include but are not limited to: 2'-F, 2'-OCH₃ ("OMe" or "O-methyl"), and 2'-O(CH₂)₂OCH₃ ("MOE"). 2'-substituent groups are described in Cook et al., U.S. 6,531,584; Cook et al., U.S. 5,859,221; and Cook et al., U.S. 6,005,087. Examples of 4'-substituent groups suitable for non-bicyclic modified sugar moieties include but are not limited to alkoxy (*e.g*., methoxy), alkyl, and those described in Manoharan et al., WO 2015/106128. Examples of 5'-substituent groups suitable for non-bicyclic modified sugar moieties include but are not limited to: 5'-methyl (R or S), 5'-vinyl, and 5'-methoxy. Modified sugar moieties and modified nucleosides are described in Migawa et al., WO 2008/101157 and Rajeev et al., US2013/0203836.).

Nucleosides comprising modified sugar moieties, such as non-bicyclic modified sugar moieties, may be referred to by the position(s) of the substitution(s) on the sugar moiety of the nucleoside. For example, nucleosides comprising 2'-substituted or 2-modified sugar moieties are referred to as 2'-substituted nucleosides or 2-modified nucleosides.

Certain modifed sugar moieties comprise a bridging sugar substituent that forms a second ring resulting in a bicyclic sugar moiety wherein the bicyclic sugar moiety comprises a bridge between the 4' and the 2' furanose ring atoms. Examples of such 4' to 2' bridging sugar substituents include but are not limited to: 4'-CH₂-2', 4'-(CH₂)₂-2', 4'-(CH₂)₃-2', 4'-CH₂-O-2' ("LNA"), 4'-CH₂-S-2', 4'-(CH₂)₂-O-2' ("ENA"), 4'-CH(CH₃)-O-2' (referred to as "constrained ethyl" or "cEt" when in the S configuration), 4'-CH₂-O-CH₂-2', 4'-CH₂-N(R)-2', 4'-CH(CH₂OCH₃)-O-2' ("constrained MOE" or "cMOE") and analogs thereof (*see, e.g.*, Seth et al., U.S. 7,399,845, Bhat et al., U.S. 7,569,686, Swayze et al., U.S. 7,741,457, and Swayze et al., U.S. 8,022,193), 4'-C(CH₃)(CH₃)-O-2' and analogs thereof (*see*, *e.g*.,Seth et al., U.S. 8,278,283), 4'-CH₂-N(OCH₃)-2' and analogs thereof (*see, e.g.,* Prakash et al., U.S. 8,278,425), 4'-CH₂-O-N(CH₃)-2' (*see, e.g.,* Allerson et al., U.S. 7,696,345 and Allerson et al., U.S. 8,124,745), 4'-CH₂-C(H)(CH₃)-2' *(see, e.g.,* Zhou, et al., J. Org. Chem.,2009, 74, 118-134), 4'-CH₂-C(=CH₂)-2' and analogs thereof (*see e.g.*, Seth et al., U.S. 8,278,426), 4'-C(RₐR_{b})-N(R)-O-2', 4'-C(RₐR_{b})-O-N(R)-2', 4'-CH₂-O-N(R)-2', and 4'-CH₂-N(R)-O-2', wherein each R, Rₐ, and R_{b} is, independently, H, a protecting group, or C₁-C₁₂ alkyl (*see, e.g.* Imanishi et al., U.S. 7,427,672).

Additional bicyclic sugar moieties are known in the art, see, for example: Freier et al., Nucleic Acids Research, 1997, 25(22), 4429-4443, Albaek et al., J. Org. Chem., 2006, 71, 7731-7740, Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc., 20017,129, 8362-8379; Elayadi et al., Curr. Opinion Invens. Drugs, 2001, 2, 558-561; Braasch et al., Chem. Biol., 2001, 8, 1-7; (Drum et al., Curr. Opinion Mol. Ther., 2001, 3, 239-243;Wengel et a., U.S. 7,053,207; Imanishi et al., U.S. 6,268,490; Imanishi et al. U.S. 6,770,748; Imanishi et al., U.S. RE44,779; Wengel et al., U.S. 6,794,499; Wengel et al., U.S. 6,670,461; Wengel et al., U.S. 7,034,133; Wengel et al., U.S. 8,080,644; Wengel et al., U.S. 8,034,909; Wengel et al., U.S. 8,153,365; Wengel et al., U.S. 7,572,582; and Ramasamy et al., U.S. 6,525,191;; Torsten et al., WO 2004/106356;Wengel et al., WO 1999/014226; Seth et al., WO 2007/134181; Seth et al., U.S. 7,547,684; Seth et al., U.S. 7,666,854; Seth et al., U.S. 8,088,746; Seth et al., U.S. 7,750,131; Seth et al., U.S. 8,030,467; Seth et al., U.S. 8,268,980; Seth et al., U.S. 8,546,556; Seth et al., U.S. 8,530,640; Migawa et al., U.S. 9,012,421; Seth et al., U.S. 8,501,805; and U.S. Patent Publication Nos. Allerson et al., US2008/0039618 and Migawa et al., US2015/0191727..

Bicyclic sugar moieties and nucleosides incorporating such bicyclic sugar moieties are further defined by isomeric configuration. For example, an LNA nucleoside (described herein) may be in the α-L configuration or in the β-D configuration. 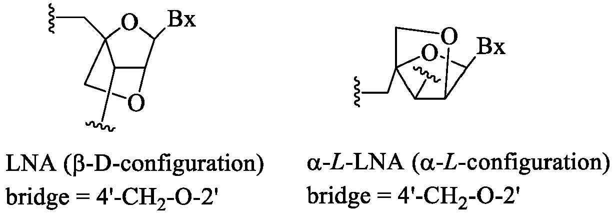 α-L-methyleneoxy (4'-CH₂-O-2') or α-L-LNA bicyclic nucleosides have been incorporated into antisense oligonucleotides that showed antisense activity (Frieden et al., Nucleic Acids Research, 2003, 21, 6365-6372). Herein, general descriptions of bicyclic nucleosides include both isomeric configurations. When the positions of specific bicyclic nucleosides (*e.g*., LNA or cEt) are identified in exemplified embodiments herein, they are in the β-D configuration, unless otherwise specified.

Certain sugar surrogates comprise a 4'-sulfur atom and a substitution at the 2'-position (*see, e.g.,* Bhat et al., U.S. 7,875,733 and Bhat et al., U.S. 7,939,677) and/or the 5' position.

Nucleosides comprising modified tetrahydropyrans include but are not limited to hexitol nucleic acid ("HNA"), anitol nucleic acid ("ANA"), manitol nucleic acid ("MNA") (*see, e.g.,* Leumann, CJ. Bioorg. & Med. Chem. 2002, 10, 841-854), fluoro HNA: 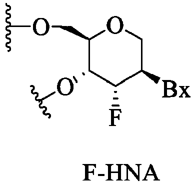 ("F-HNA", *see e.g*.Swayze et al., U.S. 8,088,904; Swayze et al., U.S. 8,440,803; Swayze et al., U.S. 8,796,437; and Swayze et al., U.S. 9,005,906; F-HNA can also be referred to as a F-THP or 3'-fluoro tetrahydropyran).

Nucleosides comprising morpholino sugar moieties and their use in oligonucleotides have been reported (*see, e.g.,* Braasch et al., Biochemistry, 2002, 41, 4503-4510 and Summerton et al., U.S. 5,698,685; Summerton et al., U.S. 5,166,315; Summerton et al., U.S. 5,185,444; and Summerton et al., U.S. 5,034,506). As used here, the term "morpholino" means a sugar surrogate having the following structure:

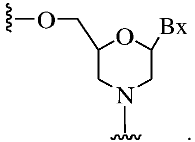

Examples of nucleosides and oligonucleotides comprising acyclic sugar surrogates include but are not limited to: peptide nucleic acid ("PNA"), acyclic butyl nucleic acid (*see, e.g.,* Kumar et al., Org. Biomol. Chem., 2013, 11, 5853-5865), and nucleosides and oligonucleotides described in Manoharan et al., WO2011/133876.

Many other bicyclic and tricyclic sugar and sugar surrogate ring systems are known in the art that can be used in modified nucleosides).

### 2. Certain Modified Nucleobases

Nucleobases include those disclosed in Merigan et al., U.S. 3,687,808, those disclosed in The Concise Encyclopedia Of Polymer Science And Engineering, Kroschwitz, J.I., Ed., John Wiley & Sons, 1990, 858-859; Englisch et al., Angewandte Chemie, International Edition, 1991, 30, 613; Sanghvi, Y.S., Chapter 15, Antisense Research and Applications, Crooke, S.T. and Lebleu, B., Eds., CRC Press, 1993, 273-288; and those disclosed in Chapters 6 and 15, Antisense Drug Technology, Crooke S.T., Ed., CRC Press, 2008, 163-166 and 442-443.

Publications that teach the preparation of certain of the above noted modified nucleobases as well as other modified nucleobases include without limitation, Manohara et al., US2003/0158403; Manoharan et al., US2003/0175906;; Dinh et al., U.S. 4,845,205; Spielvogel et al., U.S. 5,130,302; Rogers et al., U.S. 5,134,066; Bischofberger et al., U.S. 5,175,273; Urdea et al., U.S. 5,367,066; Benner et al., U.S. 5,432,272; Matteucci et al., U.S. 5,434,257; Gmeiner et al., U.S. 5,457,187; Cook et al., U.S. 5,459,255; Froehler et al., U.S. 5,484,908; Matteucci et al., U.S. 5,502,177; Hawkins et al., U.S. 5,525,711; Haralambidis et al., U.S. 5,552,540; Cook et al., U.S. 5,587,469; Froehler et al., U.S. 5,594,121; Switzer et al., U.S. 5,596,091; Cook et al., U.S. 5,614,617; Froehler et al., U.S. 5,645,985; Cook et al., U.S. 5,681,941; Cook et al., U.S. 5,811,534; Cook et al., U.S. 5,750,692; Cook et al., U.S. 5,948,903; Cook et al., U.S. 5,587,470; Cook et al., U.S. 5,457,191; Matteucci et al., U.S. 5,763,588; Froehler et al., U.S. 5,830,653; Cook et al., U.S. 5,808,027; Cook et al., 6,166,199; and Matteucci et al., U.S. 6,005,096.

### B. Certain Modified Internucleoside Linkages

The two main classes of internucleoside linking groups are defined by the presence or absence of a phosphorus atom. Representative phosphorus-containing internucleoside linkages include but are not limited to phosphates, which contain a phosphodiester bond ("P=O") (also referred to as unmodified or naturally occurring linkages), phosphotriesters, methylphosphonates, phosphoramidates, and phosphorothioates ("P=S"), and phosphorodithioates ("HS-P=S"). Representative non-phosphorus containing internucleoside linking groups include but are not limited to methylenemethylimino (-CH₂-N(CH₃)-O-CH₂-), thiodiester , thionocarbamate (-O-C(=O)(NH)-S-); siloxane (-O-SiH₂-O-); and N,N'-dimethylhydrazine (-CH₂-N(CH₃)-N(CH₃)-). Modified internucleoside linkages, compared to naturally occurring phosphate linkages, can be used to alter, typically increase, nuclease resistance of the oligonucleotide. Internucleoside linkages having a chiral atom can be prepared as a racemic mixture, or as separate enantiomers. Representative chiral internucleoside linkages include but are not limited to alkylphosphonates and phosphorothioates. Methods of preparation of phosphorous-containing and non-phosphorous-containing internucleoside linkages are well known to those skilled in the art.

Neutral internucleoside linkages include, without limitation, phosphotriesters, methylphosphonates, MMI (3'-CH₂-N(CH₃)-O-5'), amide-3 (3'-CH₂-C(=O)-N(H)-5'), amide-4 (3'-CH₂-N(H)-C(=O)-5'), formacetal (3'-O-CH₂-O-5'), methoxypropyl, and thioformacetal (3'-S-CH₂-O-5'). Further neutral internucleoside linkages include nonionic linkages comprising siloxane (dialkylsiloxane), carboxylate ester, carboxamide, sulfide, sulfonate ester and amides (See for example: Carbohydrate Modifications in Antisense Research; Y.S. Sanghvi and P.D. Cook, Eds., ACS Symposium Series 580; Chapters 3 and 4, 40-65). Further neutral internucleoside linkages include nonionic linkages comprising mixed N, O, S and CH₂ component parts.

### II. Certain Oligomeric Compounds

Conjugate groups consist of one or more conjugate moiety and a conjugate linker which links the conjugate moiety to the oligonucleotide. Conjugate groups may be attached to either or both ends of an oligonucleotide and/or at any internal position.

Examples of terminal groups include but are not limited to conjugate groups, capping groups, phosphate moieties, protecting groups, abasic nucleosides, modified or unmodified nucleosides, and two or more nucleosides that are independently modified or unmodified.

### A. Certain Conjugate Groups

Certain conjugate groups and conjugate moieties have been described previously, for example: cholesterol moiety (Letsinger et al., Proc. Natl. Acad. Sci. USA, 1989, 86, 6553-6556), cholic acid (Manoharan et al., Bioorg. Med. Chem. Lett., 1994, 4, 1053-1060), a thioether, *e.g.,* hexyl-S-tritylthiol (Manoharan et al., Ann. N.Y. Acad. Sci., 1992, 660, 306-309; Manoharan et al., Bioorg. Med. Chem. Lett., 1993, 3, 2765-2770), a thiocholesterol (Oberhauser et al., Nucl. Acids Res., 1992, 20, 533-538), an aliphatic chain, *e.g.,* do-decan-diol or undecyl residues (Saison-Behmoaras et al., EMBO J., 1991, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethyl-ammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid a palmityl moiety (Mishra et al., Biochim. Biophys. Acta, 1995, 1264, 229-237), an octadecylamine or hexylamino-carbonyl-oxycholesterol moiety (Crooke et al., J. Pharmacol. Exp. Ther., 1996, 277, 923-937), a tocopherol group (Nishina et al., Molecular Therapy Nucleic Acids, 2015, 4, e220; and Nishina et al., Molecular Therapy, 2008, 16, 734-740), or a GalNAc cluster *(e.g.,* WO2014/179620).

### 1. Conjugate Moietie

Conjugate moieties include, without limitation, intercalators, reporter molecules, polyamines, polyamides, peptides, carbohydrates (e.g., GalNAc), vitamin moieties, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins, fluorophores, and dyes.

### 2. Conjugate linkers

Conjugate moieties are attached to oligonucleotides through conjugate linkers. In certain oligomeric compounds, the conjugate linker is a single chemical bond (i.e., the conjugate moiety is attached directly to an oligonucleotide through a single bond). In certain oligomeric compounds, a conjugate moiety is attached to an oligonucleotide via a more complex conjugate linker comprising one or more conjugate linker moieities, which are sub-units making up a conjugate linker.

In general, a bifunctional linking moiety comprises at least two functional groups. One of the functional groups is selected to bind to a particular site on a parent compound and the other is selected to bind to a conjugate group. Examples of functional groups used in a bifunctional linking moiety include but are not limited to electrophiles for reacting with nucleophilic groups and nucleophiles for reacting with electrophilic groups.

Examples of conjugate linkers include but are not limited to pyrrolidine, 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other conjugate linkers include but are not limited to substituted or unsubstituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.
It is typically desirable for linker-nucleosides to be cleaved from the oligomeric compound after it reaches a target tissue. Accordingly, linker-nucleosides are typically linked to one another and to the remainder of the oligomeric compound through cleavable bonds.

Herein, linker-nucleosides are not considered to be part of the oligonucleotide. Accordingly, in embodiments in which an oligomeric compound comprises an oligonucleotide consisting of a specified number or range of linked nucleosides and/or a specified percent complementarity to a reference nucleic acid and the oligomeric compound also comprises a conjugate group comprising a conjugate linker comprising linker-nucleosides, those linker-nucleosides are not counted toward the length of the oligonucleotide and are not used in determining the percent complementarity of the oligonucleotide for the reference nucleic acid. For example, an oligomeric compound may comprise (1) a modified oligonucleotide consisting of 8-30 nucleosides and (2) a conjugate group comprising 1-10 linker-nucleosides that are contiguous with the nucleosides of the modified oligonucleotide. The total number of contiguous linked nucleosides in such an oligomeric compound is more than 30. Alternatively, an oligomeric compound may comprise a modified oligonucleotide consisting of 8-30 nucleosides and no conjugate group. The total number of contiguous linked nucleosides in such an oligomeric compound is no more than 30. Unless otherwise indicated conjugate linkers comprise no more than 10 linker-nucleosides.

For example, in certain circumstances oligomeric compounds comprising a particular conjugate moiety are better taken up by a particular cell type, but once the oligomeric compound has been taken up, it is desirable that the conjugate group be cleaved to release the unconjugated or parent oligonucleotide. Thus, certain conjugate linkers may comprise one or more cleavable moieties.

### 3. Certain Cell-Targeting Conjugate Moietiess

The conjugate group may comprise a carbohydrate cluster (*see,* e.g., Maier et al., "Synthesis of Antisense Oligonucleotides Conjugated to a Multivalent Carbohydrate Cluster for Cellular Targeting," Bioconjugate Chemistry, 2003, 14, 18-29 or Rensen et al., "Design and Synthesis of Novel N-Acetylgalactosamine-Terminated Glycolipids for Targeting of Lipoproteins to the Hepatic Asiaglycoprotein Receptor," J. Med. Chem. 2004, 47, 5798-5808). Amino sugars may be selected from any number of compounds known in the art, such as sialic acid, α-D-galactosamine, β-muramic acid, 2-deoxy-2-methylamino-L-glucopyranose, 4,6-dideoxy-4-formamido-2,3-di-*O*-methyl-D-mannopyranose, 2-deoxy-2-sulfoamino-D-glucopyranose and *N*-sulfo-D-glucosamine, and *N*-glycoloyl-α-neuraminic acid. Thio sugars may be selected from 5-Thio-β-D-glucopyranose, methyl 2,3,4-tri-*O*-acetyl-1-thio-6-*O*-trityl-α-D-glucopyranoside, 4-thio-β-D-galactopyranose, and ethyl 3,4,6,7-tetra-*O*-acetyl-2-deoxy-1,5-dithio-α-D-*gluco*-heptopyranoside.

Representative United States patents, United States patent application publications, international patent application publications, and other publications that teach the preparation of certain of the above noted conjugate groups, oligomeric compounds comprising conjugate groups, tethers, conjugate linkers, branching groups, ligands, cleavable moieties as well as other modifications include without limitation, US 5,994,517, US 6,300,319, US 6,660,720, US 6,906,182, US 7,262,177, US 7,491,805, US 8,106,022, US 7,723,509, US 2006/0148740, US 2011/0123520, WO 2013/033230 and WO 2012/037254, Biessen et al., J. Med. Chem. 1995, 38, 1846-1852, Lee et al., Bioorganic & Medicinal Chemistry 2011,19, 2494-2500, Rensen et al., J. Biol. Chem. 2001, 276, 37577-37584, Rensen et al., J. Med. Chem. 2004, 47, 5798-5808, Sliedregt et al., J. Med. Chem. 1999, 42, 609-618, and Valentijn et al., Tetrahedron, 1997, 53, 759-770.

Antisense compounds and oligomeric compounds may comprise a conjugate group found in any of the following references: Lee, Carbohydr Res, 1978, 67, 509-514; Connolly et al., J Biol Chem, 1982, 257, 939-945; Pavia et al., Int J Pep Protein Res, 1983, 22, 539-548; Lee et al., Biochem, 1984, 23, 4255-4261; Lee et al., Glycoconjugate J, 1987, 4, 317-328; Toyokuni et al., Tetrahedron Lett, 1990, 31, 2673-2676; Biessen et al., J Med Chem, 1995, 38, 1538-1546; Valentijn et al., Tetrahedron, 1997, 53, 759-770; Kim et al., Tetrahedron Lett, 1997, 38, 3487-3490; Lee et al., Bioconjug Chem, 1997, 8, 762-765; Kato et al., Glycobiol, 2001, 11, 821-829; Rensen et al., J Biol Chem, 2001, 276, 37577-37584; Lee et al., Methods Enzymol, 2003, 362, 38-43; Westerlind et al., Glycoconj J, 2004, 21, 227-241; Lee et al., Bioorg Med Chem Lett, 2006, 16(19), 5132-5135; Maierhofer et al., Bioorg Med Chem, 2007, 15, 7661-7676; Khorev et al., Bioorg Med Chem, 2008, 16, 5216-5231; Lee et al., Bioorg Med Chem, 2011, 19, 2494-2500; Kornilova et al., Analyt Biochem, 2012, 425, 43-46; Pujol et al., Angew Chemie Int Ed Engl, 2012, 51, 7445-7448; Biessen et al., J Med Chem, 1995, 38, 1846-1852; Sliedregt et al., J Med Chem, 1999, 42, 609-618; Rensen et al., J Med Chem, 2004, 47, 5798-5808; Rensen et al., Arterioscler Thromb Vasc Biol, 2006, 26, 169-175; van Rossenberg et al., Gene Ther, 2004, 11, 457-464; Sato et al., J Am Chem Soc, 2004, 126, 14013-14022; Lee et al., J Org Chem, 2012, 77, 7564-7571; Biessen et al., FASEB J, 2000, 14, 1784-1792; Rajur et al., Bioconjug Chem, 1997, 8, 935-940; Duff et al., Methods Enzymol, 2000, 313, 297-321; Maier et al., Bioconjug Chem, 2003, 14, 18-29; Jayaprakash et al., Org Lett, 2010, 12, 5410-5413; Manoharan, Antisense Nucleic Acid Drug Dev, 2002, 12, 103-128; Merwin et al., Bioconjug Chem, 1994, 5, 612-620; Tomiya et al., Bioorg Med Chem, 2013, 21, 5275-5281; International applications WO1998/013381; WO2011/038356; WO1997/046098; WO2008/098788; WO2004/101619; WO2012/037254; WO2011/120053; WO2011/100131; WO2011/163121; WO2012/177947; WO2013/033230; WO2013/075035; WO2012/083185; WO2012/083046; WO2009/082607; WO2009/134487; WO2010/144740; WO2010/148013; WO1997/020563; WO2010/088537; WO2002/043771; WO2010/129709; WO2012/068187; WO2009/126933; WO2004/024757; WO2010/054406; WO2012/089352; WO2012/089602; WO2013/166121; WO2013/165816; U.S. Patents 4,751,219; 8,552,163; 6,908,903; 7,262,177; 5,994,517; 6,300,319; 8,106,022; 7,491,805; 7,491,805; 7,582,744; 8,137,695; 6,383,812; 6,525,031; 6,660,720; 7,723,509; 8,541,548; 8,344,125; 8,313,772; 8,349,308; 8,450,467; 8,501,930; 8,158,601; 7,262,177; 6,906,182; 6,620,916; 8,435,491; 8,404,862; 7,851,615; Published U.S. Patent Application Publications US2011/0097264; US2011/0097265; US2013/0004427; US2005/0164235; US2006/0148740; US2008/0281044; US2010/0240730; US2003/0119724; US2006/0183886; US2008/0206869; US2011/0269814; US2009/0286973; US2011/0207799; US2012/0136042; US2012/0165393; US2008/0281041; US2009/0203135; US2012/0035115; US2012/0095075; US2012/0101148; US2012/0128760; US2012/0157509; US2012/0230938; US2013/0109817; US2013/0121954; US2013/0178512; US2013/0236968; US2011/0123520; US2003/0077829; US2008/0108801; and US2009/0203132.

### III. Certain Antisense Compounds

Such selective antisense compounds comprises a nucleobase sequence that hybridizes to one or more target nucleic acid, resulting in one or more desired antisense activity and does not hybridize to one or more non-target nucleic acid or does not hybridize to one or more non-target nucleic acid in such a way that results in significant undesired antisense activity.

In certain antisense activities, hybridization of an antisense compound to a target nucleic acid results in recruitment of a protein that cleaves the target nucleic acid. For example, certain antisense compounds result in RNase H mediated cleavage of the target nucleic acid. RNase H is a cellular endonuclease that cleaves the RNA strand of an RNA:DNA duplex. The DNA in such an RNA:DNA duplex need not be unmodified DNA.

In certain antisense activities, an antisense compound or a portion of an antisense compound is loaded into an RNA-induced silencing complex (RISC), ultimately resulting in cleavage of the target nucleic acid. For example, certain antisense compounds result in cleavage of the target nucleic acid by Argonaute. Antisense compounds that are loaded into RISC are RNAi compounds. RNAi compounds may be double-stranded (siRNA) or single-stranded (ssRNA).

Antisense activities may be observed directly or indirectly. Observation or detection of an antisense activity involves observation or detection of a change in an amount of a target nucleic acid or protein encoded by such target nucleic acid, a change in the ratio of splice variants of a nucleic acid or protein, and/or a phenotypic change in a cell or animal.

### B. Apolipoprotein (a) (apo(a))

In certain embodiments useful for understanding the invention, conjugated antisense compounds target any apo(a) nucleic acid. In certain embodiments useful for understanding the invention, the target nucleic acid encodes an apo(a) target protein that is clinically relevant. In such embodiments, useful for understanding the invention, modulation of the target nucleic acid results in clinical benefit.

The targeting process usually includes determination of at least one target region, segment, or site within the target nucleic acid for the antisense interaction to occur such that the desired effect will result.

One apo(a) protein is linked via a disulfide bond to a single apolipoprotein B (apoB) protein to form a lipoprotein(a) (Lp(a)) particle. The apo(a) protein shares a high degree of homology with plasminogen particularly within the kringle IV type 2 repetitive domain. It is thought that the kringle repeat domain in apo(a) may be responsible for its pro-thrombotic and anti-fibrinolytic properties, potentially enhancing atherosclerotic progression. Apo(a) is transcriptionally regulated by IL-6 and in studies in rheumatoid arthritis patients treated with an IL-6 inhibitor (tocilizumab), plasma levels were reduced by 30% after 3 month treatment. Apo(a) has been shown to preferentially bind oxidized phospholipids and potentiate vascular inflammation. Further, studies suggest that the Lp(a) particle may also stimulate endothelial permeability, induce plasminogen activator inhibitor type-1 expression and activate macrophage interleukin-8 secretion. Importantly, recent genetic association studies revealed that Lp(a) was an independent risk factor for myocardial infarction, stroke, peripheral vascular disease and abdominal aortic aneurysm. Further, in the Precocious Coronary Artery Disease (PROCARDIS) study, Clarke *et al.* described robust and independent associations between coronary heart disease and plasma Lp(a) concentrations. Additionally, Solfrizzi et al., suggested that increased serum Lp(a) may be linked to an increased risk for Alzheimer's Disease (AD). Antisense compounds targeting apo(a) have been previously disclosed in WO2005/000201 and US2010-0331390. An antisense oligonucleobase targeting Apo(a), ISIS-APOA_{Rx}, was assessed in a Phase I clinical trial to study it's safety profile.

### Certain Conjugated Antisense Compounds Targeted to an Apo(a) Nucleic Acid

In certain embodiments useful for understanding the invention, conjugated antisense compounds are targeted to an Apo(a) nucleic acid having the sequence of GENBANK^{®} Accession No. NM_005577.2, incorporated herein as SEQ ID NO: 1; GENBANK Accession No. NT_007422.12 truncated from nucleotides 3230000 to 3380000, incorporated herein as SEQ ID NO: 2; GENBANK Accession No. NT_025741.15 truncated from nucleotides 65120000 to 65258000, designated herein as SEQ ID NO: 3; and GENBANK Accession No. NM_005577.1, incorporated herein as SEQ ID NO: 4. In certain such embodiments useful for understanding the invention, a conjugated antisense compound is at least 90%, at least 95%, or 100% complementary to any of the nucleobase sequences of SEQ ID NOs: 1-4.

**Table A: Antisense Compounds targeted to Apo(a) SEQ ID NO: 1**

| **ISIS No** | **Target Start Site** | Sequence (5'-3') | **Motif** | **SEQ ID NO** |
|---|---|---|---|---|
| 494372 | 3901 | TGCTCCGTTGGTGCTTGTTC | eeeeeddddddddddeeeee | 7 |
| 494283 | 584 | TCTTCCTGTGACAGTGGTGG | eeeeeddddddddddeeeee | 8 |
| | 926 | | | |
| | 1610 | | | |
| | 1952 | | | |
| | 2294 | | | |
| | 3320 | | | |
| 494284 | 585 | TTCTTCCTGTGACAGTGGTG | eeeeeddddddddddeeeee | 9 |
| | 927 | | | |
| | 1611 | | | |
| | 1953 | | | |
| | 2295 | | | |
| | 3321 | | | |
| 494286 | 587 | GGTTCTTCCTGTGACAGTGG | eeeeeddddddddddeeeee | 10 |
| | 929 | | | |
| | 1613 | | | |
| | 1955 | | | |
| | 2297 | | | |
| 494301 | 628 | CGACTATGCGAGTGTGGTGT | eeeeeddddddddddeeeee | 11 |
| | 970 | | | |
| | 1312 | | | |
| | 1654 | | | |
| | 1996 | | | |
| | 2338 | | | |
| | 2680 | | | |
| | 3022 | | | |
| 494302 | 629 | CCGACTATGCGAGTGTGGTG | eeeeeddddddddddeeeee | 12 |
| | 971 | | | |
| | 1313 | | | |
| | 1655 | | | |
| | 1997 | | | |
| | 2339 | | | |
| | 2681 | | | |
| | 3023 | | | |

In certain embodiments useful for understanding the invention, the present disclosure provides conjugated antisense compounds represented by the following structure. This structure corresponds to ISIS 681257. In certain embodiments useful for understanding the invention, the antisense compound comprises the conjugated modified oligonucleotide ISIS 681257 or a salt thereof. In certain embodiments useful for understanding the invention, the antisense compound consists of the conjugated modified oligonucleotide ISIS 681257. However, it must be made clear that ISIS 681257 does not form part of the invention.

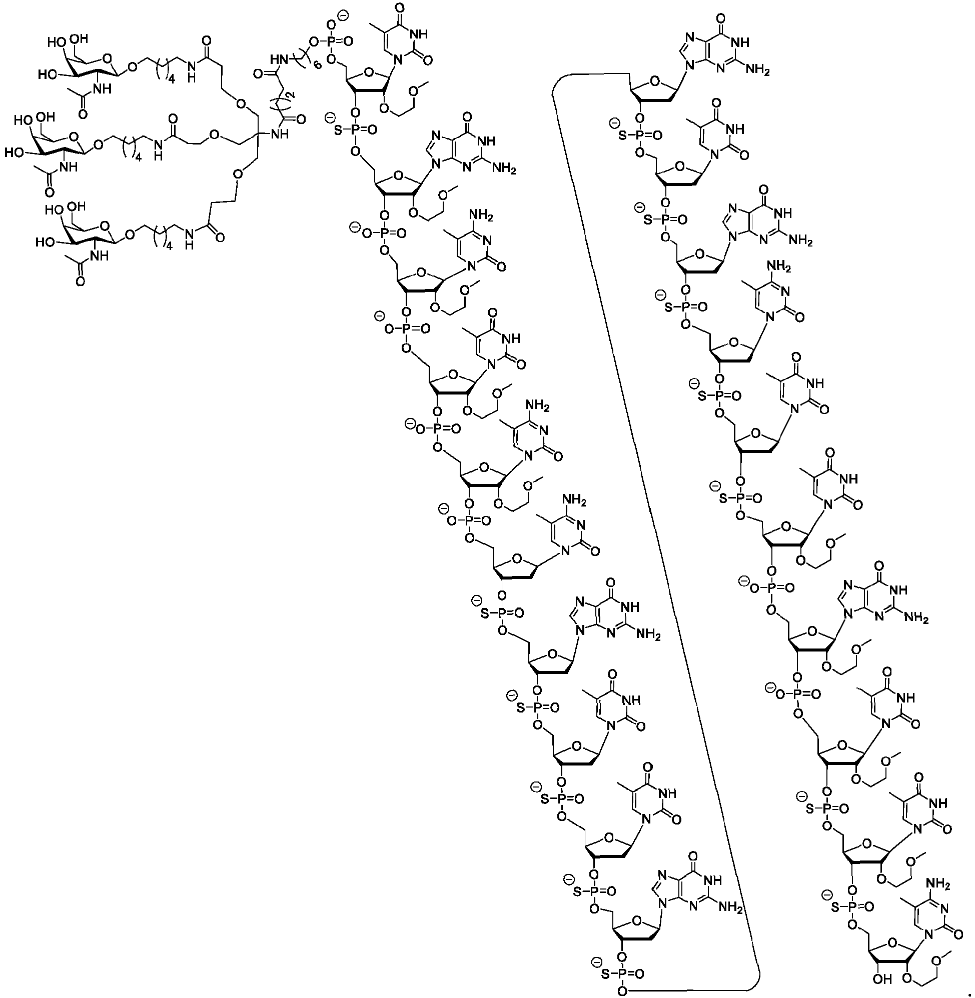

### Apo(a) Therapeutic Indications

In certain embodiments useful for understanding the invention, the invention provides methods for using a conjugated antisense compound targeted to an apo(a) nucleic acid for modulating the expression of apo(a) in a subject. In certain embodiments useful for understanding the invention, the expression of apo(a) is reduced.

In certain embodiments useful for understanding the invention, provided herein are methods of treating a subject comprising administering one or more pharmaceutical compositions as described herein. In certain embodiments useful for understanding the invention, the invention provides methods for using a conjugated antisense compound targeted to an apo(a) nucleic acid in a pharmaceutical composition for treating a subject. In certain embodiments useful for understanding the invention, the individual has an apo(a) related disease. In certain embodiments useful for understanding the invention, the individual has an Lp(a) related disease. In certain embodiments useful for understanding the invention, the individual has an inflammatory, cardiovascular and/or a metabolic disease, disorder or condition.

In certain embodiments useful for understanding the invention, the subject has an inflammatory, cardiovascular and/or metabolic disease, disorder or condition.

In certain embodiments useful for understanding the invention, the cardiovascular diseases, disorders or conditions (CVD) include, but are not limited to, elevated Lp(a) associated CVD risk, recurrent cardiovascular events with elevated Lp(a), aortic stenosis (e.g., calcific aortic stenosis associated with elevated Lp(a)), aneurysm (e.g., abdominal aortic aneurysm), angina, arrhythmia, atherosclerosis, cerebrovascular disease, coronary artery disease, coronary heart disease, dyslipidemia, hypercholesterolemia, hyperlipidemia, hypertension, hypertriglyceridemia, myocardial infarction, peripheral vascular disease (e.g., peripheral artery disease), stroke and the like.

In certain embodiments useful for understanding the invention, the compounds targeted to apo(a) described herein modulate physiological markers or phenotypes of the cardiovascular disease, disorder or condition. For example, administration of the compounds to animals can decrease Lp(a), LDL and cholesterol levels in those animals compared to untreated animals. In certain embodiments useful for understanding the invention, the modulation of the physiological markers or phenotypes can be associated with inhibition of apo(a) by the compounds.

In certain embodiments useful for understanding the invention, the physiological markers of the cardiovascular disease, disorder or condition can be quantifiable. For example, Lp(a), LDL or cholesterol levels can be measured and quantified by, for example, standard lipid tests. For such markers, in certain embodiments useful for understanding the invention, the marker can be decreased by about 5, 10, 15, 20, 25, 30, 35, 40, 45, 50, 55, 60, 65, 70, 75, 80, 85, 90, 95 or 99%, or a range defined by any two of these values.

Also, provided herein are methods for preventing, treating or ameliorating a symptom associated with the cardiovascular disease, disorder or condition in a subject in need thereof. In certain embodiments useful for understanding the invention, provided is a method for reducing the rate of onset of a symptom associated with the cardiovascular disease, disorder or condition. In certain embodiments useful for understanding the invention, provided is a method for reducing the severity of a symptom associated with the cardiovascular disease, disorder or condition. In such embodiments useful for understanding the invention, the methods comprise administering a therapeutically effective amount of a compound targeted to an apo(a) nucleic acid to an individual in need thereof.

The cardiovascular disease, disorder or condition can be characterized by numerous physical symptoms. Any symptom known to one of skill in the art to be associated with the cardiovascular disease, disorder or condition can be prevented, treated, ameliorated or otherwise modulated with the compounds and methods described herein. In certain embodiments useful for understanding the invention, the symptom can be any of, but not limited to, angina, chest pain, shortness of breath, palpitations, weakness, dizziness, nausea, sweating, tachycardia, bradycardia, arrhythmia, atrial fibrillation, swelling in the lower extremities, cyanosis, fatigue, fainting, numbness of the face, numbness of the limbs, claudication or cramping of muscles, bloating of the abdomen or fever.

In certain embodiments useful for understanding the invention, the metabolic diseases, disorders or conditions include, but are not limited to, hyperglycemia, prediabetes, diabetes (type I and type II), obesity, insulin resistance, metabolic syndrome and diabetic dyslipidemia.

In certain embodiments useful for understanding the invention, compounds targeted to apo(a) as described herein modulate physiological markers or phenotypes of the metabolic disease, disorder or condition. For example, administrion of the compounds to animals can decrease glucose and insulin resistance levels in those animals compared to untreated animals. In certain embodiments useful for understanding the invention, the modulation of the physiological markers or phenotypes can be associated with inhibition of apo(a) by the compounds.

In certain embodiments useful for understanding the invention, physiological markers of the metabolic disease, disorder or condition can be quantifiable. For example, glucose levels or insulin resistance can be measured and quantified by standard tests known in the art. For such markers, in certain embodiments useful for understanding the invention, the marker can be decreased by about 5, 10, 15, 20, 25, 30, 35, 40, 45, 50, 55, 60, 65, 70, 75, 80, 85, 90, 95 or 99%, or a range defined by any two of these values. In another example, insulin sensitivity can be measured and quantified by standard tests known in the art. For such markers, in certain embodiments useful for understanding the invention, the marker can be increase by about 5, 10, 15, 20, 25, 30, 35, 40, 45, 50, 55, 60, 65, 70, 75, 80, 85, 90, 95 or 99%, or a range defined by any two of these values.

Also, provided herein are methods for preventing, treating or ameliorating a symptom associated with the metabolic disease, disorder or condition in a subject in need thereof. In certain embodiments useful for understanding the invention, provided is a method for reducing the rate of onset of a symptom associated with the metabolic disease, disorder or condition. In certain embodiments useful for understanding the invention, provided is a method for reducing the severity of a symptom associated with the metabolic disease, disorder or condition. In such embodiments useful for understanding the invention, the methods comprise administering a therapeutically effective amount of a compound targeted to an apo(a) nucleic acid to an individual in need thereof.

The metabolic disease, disorder or condition can be characterized by numerous physical symptoms. Any symptom known to one of skill in the art to be associated with the metabolic disease, disorder or condition can be prevented, treated, ameliorated or otherwise modulated with the compounds and methods described herein. In certain embodiments useful for understanding the invention, the symptom can be any of, but not limited to, excessive urine production (polyuria), excessive thirst and increased fluid intake (polydipsia), blurred vision, unexplained weight loss and lethargy.

In certain embodiments useful for understanding the invention, the inflammatory diseases, disorders or conditions include, but are not limited to, elevated Lp(a) associated CVD risk, recurrent cardiovascular events with elevated Lp(a), aortic stenosis (e.g., calcific aortic valve stenosis associated with high Lp(a)), coronary artey disease (CAD), Alzheimer's Disease and thromboembolic diseases, disorder or conditions. Certain thromboembolic diseases, disorders or conditions include, but are not limited to, stroke, thrombosis, myocardial infarction and peripheral vascular disease.

In certain embodiments useful for understanding the invention, the compounds targeted to apo(a) described herein modulate physiological markers or phenotypes of the inflammatory disease, disorder or condition. For example, administration of the compounds to animals can decrease inflammatory cytokine or other inflammatory markers levels in those animals compared to untreated animals. In certain embodiments useful for understanding the invention, the modulation of the physiological markers or phenotypes can be associated with inhibition of apo(a) by the compounds.

In certain embodiments useful for understanding the invention, the physiological markers of the inflammatory disease, disorder or condition can be quantifiable. For example, cytokine levels can be measured and quantified by standard tests known in the art. For such markers, in certain embodiments useful for understanding the invention, the marker can be decreased by at least about 5%, 10%, 15%, 20%, 25%, 30%, 35%, 40%, 45%, 50%, 55%, 60%, 65%, 70%, 75%, 80%, 85%, 90%, 95% or 99%, or a range defined by any two of these values.

Also, provided herein are methods for preventing, treating or ameliorating a symptom associated with the inflammatory disease, disorder or condition in a subject in need thereof. In certain embodiments useful for understanding the invention, provided is a method for reducing the rate of onset of a symptom associated with the inflammatory disease, disorder or condition. In certain embodiments useful for understanding the invention, provided is a method for reducing the severity of a symptom associated with the inflammatory disease, disorder or condition. In such embodiments useful for understanding the invention, the methods comprise administering a therapeutically effective amount of a compound targeted to an apo(a) nucleic acid to an individual in need thereof.

In certain embodiments useful for understanding the invention, provided are methods of treating an individual with an apo(a) related disease, disorder or condition comprising administering a therapeutically effective amount of one or more pharmaceutical compositions as described herein. In certain embodiments useful for understanding the invention, the individual has elevated apo(a) levels. In certain embodiments useful for understanding the invention, provided are methods of treating an individual with an Lp(a) related disease, disorder or condition comprising administering a therapeutically effective amount of one or more pharmaceutical compositions as described herein. In certain embodiments useful for understanding the invention, the individual has elevated Lp(a) levels. In certain embodiments useful for understanding the invention, the individual has an inflammatory, cardiovascular and/or metabolic disease, disorder or condition. In certain embodiments useful for understanding the invention, administration of a therapeutically effective amount of an antisense compound targeted to an apo(a) nucleic acid is accompanied by monitoring of apo(a) or Lp(a) levels. In certain embodiments useful for understanding the invention, administration of a therapeutically effective amount of an antisense compound targeted to an apo(a) nucleic acid is accompanied by monitoring of markers of inflammatory, cardiovascular and/or metabolic disease, or other disease process associated with the expression of apo(a), to determine an individual's response to the antisense compound. An individual's response to administration of the antisense compound targeting apo(a) can be used by a physician to determine the amount and duration of therapeutic intervention with the compound.

In certain embodiments useful for understanding the invention, administration of an antisense compound targeted to an apo(a) nucleic acid results in reduction of apo(a) expression by at least about 15%, 20%, 25%, 30%, 35%, 40%, 45%, 50%, 55%, 60%, 65%, 70%, 75%, 80%, 85%, 90%, 95% or 99%, or a range defined by any two of these values. In certain embodiments useful for understanding the invention, apo(a) expression is reduced to at least -<- 100 mg/dL, ≤ 90 mg/dL, ≤ 80 mg/dL, ≤ 70 mg/dL, ≤ 60 mg/dL, ≤ 50 mg/dL, ≤ 40 mg/dL, ≤ 30 mg/dL, ≤20 mg/dL or ≤ 10 mg/dL.

In certain embodiments useful for understanding the invention, administration of an antisense compound targeted to an apo(a) nucleic acid results in reduction of Lp(a) expression by at least about 15%, 20%, 25%, 30%, 35%, 40%, 45%, 50%, 55%, 60%, 65%, 70%, 75%, 80%, 85%, 90%, 95% or 99%, or a range defined by any two of these values. In certain embodiments useful for understanding the invention, Lp(a) expression is reduced to at least ≤ 200 mg/dL, ≤ 190 mg/dL, ≤ 180 mg/dL, ≤ 175 mg/dL, ≤ 170 mg/dL, ≤ 160 mg/dL, ≤ 150 mg/dL, ≤ 140 mg/dL, ≤ 130 mg/dL, ≤ 120 mg/dL, ≤ 110 mg/dL, ≤ 100 mg/dL, ≤ 90 mg/dL, ≤ 80 mg/dL, ≤ 70 mg/dL, ≤ 60 mg/dL, ≤ 55 mg/dL, ≤ 50 mg/dL, ≤ 45 mg/dL, ≤ 40 mg/dL, ≤ 35 mg/dL, ≤ 30 mg/dL, ≤ 25 mg/dL, ≤ 20 mg/dL, ≤ 15 mg/dL, or ≤ 10 mg/dL.

In certain embodiments useful for understanding the invention, the disclosure provides methods for using a conjugated antisense compound targeted to an apo(a) nucleic acid in the preparation of a medicament. In certain embodiments useful for understanding the invention, pharmaceutical compositions comprising a conjugated antisense compound targeted to apo(a) are used for the preparation of a medicament for treating a patient suffering or susceptible to an inflammatory, cardiovascular and/or a metabolic disease, disorder or condition.

### Apo(a) Treatment Populations

Certain subjects with high Lp(a) levels are at a significant risk of various diseases (Lippi et al., Clinica Chimica Acta, 2011, 412:797-801; Solfrizz et al.). In many subjects with high Lp(a) levels, current treatments cannot reduce their Lp(a) levels to safe levels. Apo(a) plays an important role in the formation of Lp(a), hence reducing apo(a) can reduce Lp(a) and prevent, treat or ameliorate a disease associated with Lp(a).

In certain embodiments useful for understanding the invention, treatment with the compounds and methods disclosed herein is indicated for a human animal with elevated apo(a) levels and/or Lp(a) levels. In certain embodiments useful for understanding the invention, the human has apo(a) levels ≥ 10 mg/dL, ≥ 20 mg/dL, ≥ 30 mg/dL, ≥ 40 mg/dL, ≥ 50 mg/dL, ≥ 60 mg/dL, ≥ 70 mg/dL, ≥ 80 mg/dL, ≥ 90 mg/dL or ≥ 100 mg/dL. In certain embodiments useful for understanding the invention, the human has Lp(a) levels ≥ 10 mg/dL, ≥ 15 mg/dL, ≥ 20 mg/dL, ≥ 25 mg/dL, ≥ 30 mg/dL, ≥ 35 mg/dL, ≥ 40 mg/dL, ≥ 50 mg/dL, ≥ 60 mg/dL, ≥ 70 mg/dL, ≥ 80 mg/dL, ≥ 90 mg/dL, ≥ 100 mg/dL, ≥ 110 mg/dL, ≥ 120 mg/dL, ≥ 130 mg/dL, ≥ 140 mg/dL, ≥ 150 mg/dL, ≥ 160 mg/dL, ≥ 170 mg/dL, ≥ 175 mg/dL, ≥ 180 mg/dL, ≥ 190 mg/dL, ≥ 200 mg/dL.

### C. Apolipoprotein C-III (ApoCIII)

ApoCIII is a constituent of HDL and of triglyceride (TG)-rich lipoproteins. Elevated ApoCIII levels are associated with elevated TG levels and diseases such as cardiovascular disease, metabolic syndrome, obesity and diabetes. Elevated TG levels are associated with pancreatitis. ApoCIII slows clearance of TG-rich lipoproteins by inhibiting lipolysis through inhibition of lipoprotein lipase (LPL) and through interfering with lipoprotein binding to cell-surface glycosaminoglycan matrix. Antisense compounds targeting ApoCIII have been previously disclosed in WO2004/093783 and WO2012/149495. Currently, an antisense oligonucleotide targeting ApoCIII, ISIS-APOCIII_{Rx}, is in Phase II clinical trials to assess its effectiveness in the treatment of diabetes or hypertriglyceridemia. However, there is still a need to provide patients with additional and more potent treatment options.

### a. Certain Conjugated Antisense Compounds Targeted to an ApoCIII Nucleic Acid

In certain embodiments, conjugated antisense compounds are targeted to an ApoCIII nucleic acid having the sequence of GENBANK^{®} Accession No. NT_033899.8 truncated from nucleobases 20262640 to 20266603, incorporated herein as SEQ ID NO: 6. In certain such embodiments, a conjugated antisense compound is at least 90%, at least 95%, or 100% complementary to SEQ ID NO: 6.

In certain embodiments, conjugated antisense compounds are targeted to an ApoCIII nucleic acid having the sequence of GENBANK^{®} Accession No. NM_000040.1, incorporated herein as SEQ ID NO: 5.

The conjugated antisense compound, as defined in the appended claims, targeted to SEQ ID NO: 5 comprises a nucleobase sequence of SEQ ID NO: 13.

**Table 5: Antisense Compounds targeted to ApoCIII SEQ ID NO: 5**

| **ISIS No** | **Target Start Site** | **Sequence (5'-3')** | **Motif** | **SEQ ID NO** |
|---|---|---|---|---|
| 304801 | 508 | AGCTTCTTGTCCAGCTTTAT | eeeeeddddddddddeeeee | 13 |
| 647535 | 508 | AGCTTCTTGTCCAGCTTTAT | eeeeeddddddddddeeeeeod | 13 |
| 616468 | 508 | AGCTTCTTGTCCAGCTTTAT | eeeeeddddddddddeeeee | 13 |
| 647536 | 508 | AGCTTCTTGTCCAGCTTTAT | eeoeoeoeoddddddddddeoe oeeeod | 13 |

The present invention provides conjugated antisense compounds represented by the following structure for use as defined in the appended claims. This structure corresponds to ISIS 678354. In certain embodiments, the antisense compound comprises the conjugated modified oligonucleotide ISIS 678354 or a salt thereof. In certain embodiments, the antisense compound consists of the conjugated modified oligonucleotide ISIS 678354.

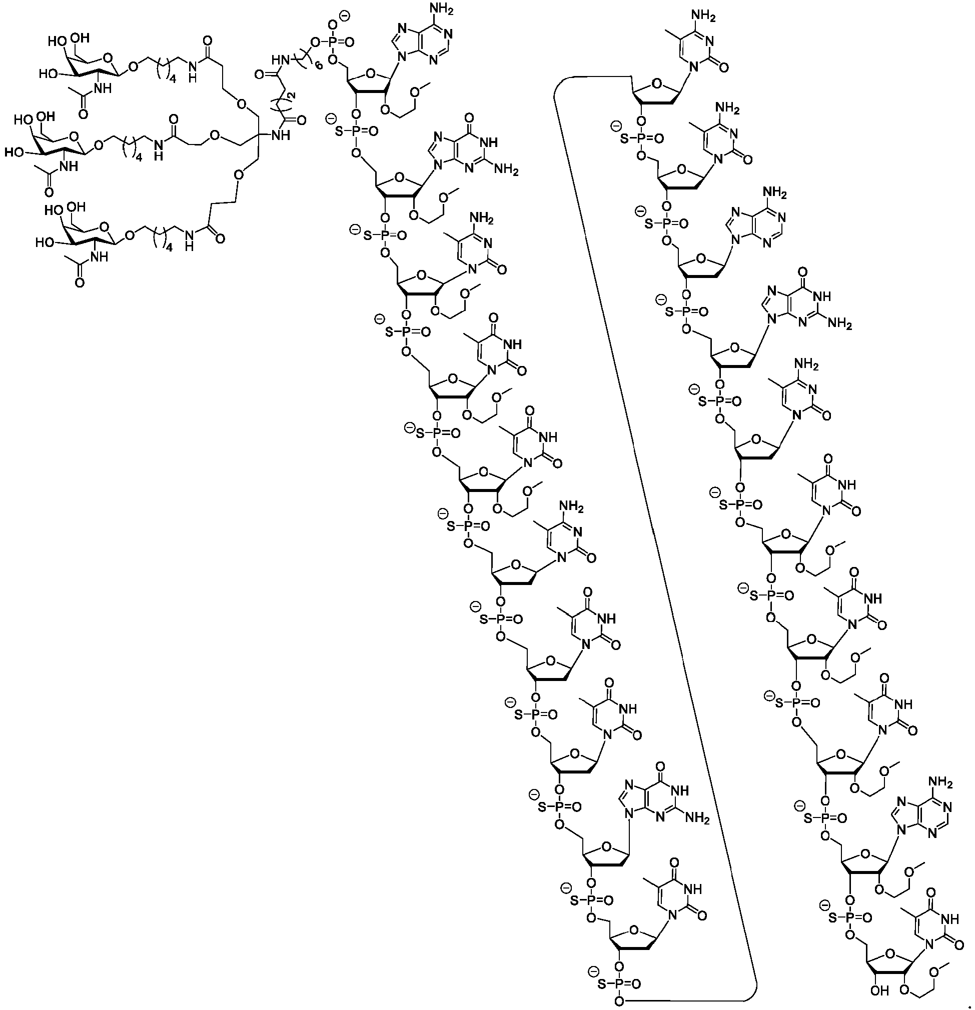

### b. ApoCIII Therapeutic Indications

In certain embodiments, the invention provides a conjugated antisense compound, as defined in the appended claims, targeted to an ApoCIII nucleic acid for use in methods for modulating the expression of ApoCIII in a subject. In certain embodiments, the expression of ApoCIII is reduced.

In certain embodiments, the invention provides a conjugated antisense compound, as defined in the appended claims, targeted to an ApoCIII nucleic acid in a pharmaceutical composition for use in methods of treating a subject. In certain embodiments, the subject has a cardiovascular and/or metabolic disease, disorder or condition. In certain embodiments, the subject has hypertriglyceridemia, non-familial hypertriglyceridemia, familial hypertriglyceridemia, heterozygous familial hypertriglyceridemia, homozygous familial hypertriglyceridemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia (e.g., lipodystrophy), hyperlipidemia, hypercholesterolemia, hyperfattyacidemia, hepatic steatosis, non-alcoholic steatohepatitis, pancreatitis and/or non-alcoholic fatty liver disease.

In certain embodiments, the invention provides a conjugated antisense compound, as defined in the appended claims, targeted to an ApoCIII nucleic acid for use in the preparation of a medicament.

### a. Certain ApoCIII Dosing Regimens

ISIS 678354 is administered to a subject in need thereof. In certain embodiments, 20 mg of ISIS 678354is administered to a human subject. In certain embodiments, 40 mg of ISIS 678354 is administered to a human subject. In certain embodiments, 80 mg of ISIS 678354 is administered to a human subject. In certain embodiments, 120 mg of ISIS 678354 is administered to a human subject.

ISIS 678354 is administered to a subject in need thereof. In certain embodiments, 20 mg of ISIS 678354 is administered to a human subject during a dosing period. In certain embodiments, 40 mg of ISIS 678354 is administered to a human subject during a dosing period. In certain embodiments, 80 mg of ISIS 678354 is administered to a human subject during a dosing period. In certain embodiments, 120 mg of ISIS 678354 is administered to a human subject during a dosing period. In certain embodiments, the dosing period is one week. In certain embodiments, only one dose is given during the dosing period.

In certain embodiments, 20 mg of ISIS 678354 is administered to a human subject each week. In certain embodiments, 40 mg of ISIS 678354 is administered to a human subject each week.

### D. Angiopoietin-Like 3

The angiopoietins are a family of secreted growth factors. Together with their respective endothelium-specific receptors, the angiopoietins play important roles in angiogenesis. One family member, angiopoietin-like 3 (also known as angiopoietin-like protein 3, ANGPT5, ANGPTL3, or angiopoietin 5), is predominantly expressed in the liver, and is thought to play a role in regulating lipid metabolism (Kaplan et al., J. Lipid Res., 2003, 44, 136-143). Genome-wide association scans (GWAS) surveying the genome for common variants associated with plasma concentrations of HDL, LDL and triglyceride found an association between triglycerides and single-nucleotide polymorphisms (SNPs) near ANGPTL3 (Willer et al., Nature Genetics, 2008, 40(2):161-169). Individuals with homozygous ANGPTL3 loss-of-function mutations present with low levels of all atherogenic plasma lipids and lipoproteins, such as total cholesterol (TC) and TG, low density lipoprotein cholesterol (LDL-C), apoliprotein B (apoB), non-HDL-C, as well as HDL-C (Romeo et al. 2009, J Clin Invest, 119(1):70-79; Musunuru et al. 2010 TV Engl J Med, 363:2220-2227; Martin-Campos et al. 2012, Clin Chim Acta, 413:552-555; Minicocci et al. 2012, J Clin Endocrinol Metab, 97:e1266-1275; Noto et al. 2012, Arterioscler Thromb Vasc Biol, 32:805-809; Pisciotta et al. 2012, Circulation Cardiovasc Genet, 5:42-50). This clinical phenotype has been termed familial combined hypolipidemia (FHBL2). Despite reduced secretion of VLDL, subjects with FHBL2 do not have increased hepatic fat content. They also appear to have lower plasma glucose and insulin levels, and importantly, both diabetes and cardiovascular disease appear to be absent from these subjects. No adverse clinical phenotypes have been reported to date (Minicocci et al. 2013, J of Lipid Research, 54:3481-3490). Reduction of ANGPTL3 has been shown to lead to a decrease in TG, cholesterol and LDL levels in animal models (U.S. Serial Number 13/520,997; PCT Publication WO 2011/085271). Mice deficient in ANGPTL3 have very low plasma triglyceride (TG) and cholesterol levels, while overpexpression produces the opposite effects (Koishi et al. 2002; Koster 2005; Fujimoto 2006). Accordingly, the potential role of ANGPTL3 in lipid metabolism makes it an attractive target for therapeutic intervention.

In certain embodiments useful for understanding the invention, the present disclosure provides conjugated antisense compounds represented by the following structure. This structure corresponds to ISIS 703802. In certain embodiments useful for understanding the invention, the antisense compound comprises the conjugated modified oligonucleotide ISIS 703802 or a salt thereof. In certain embodiments useful for understanding the invention, the antisense compound consists of the conjugated modified oligonucleotide ISIS 703802. However, it must be made clear that ISIS 703802 does not form part of the invention.

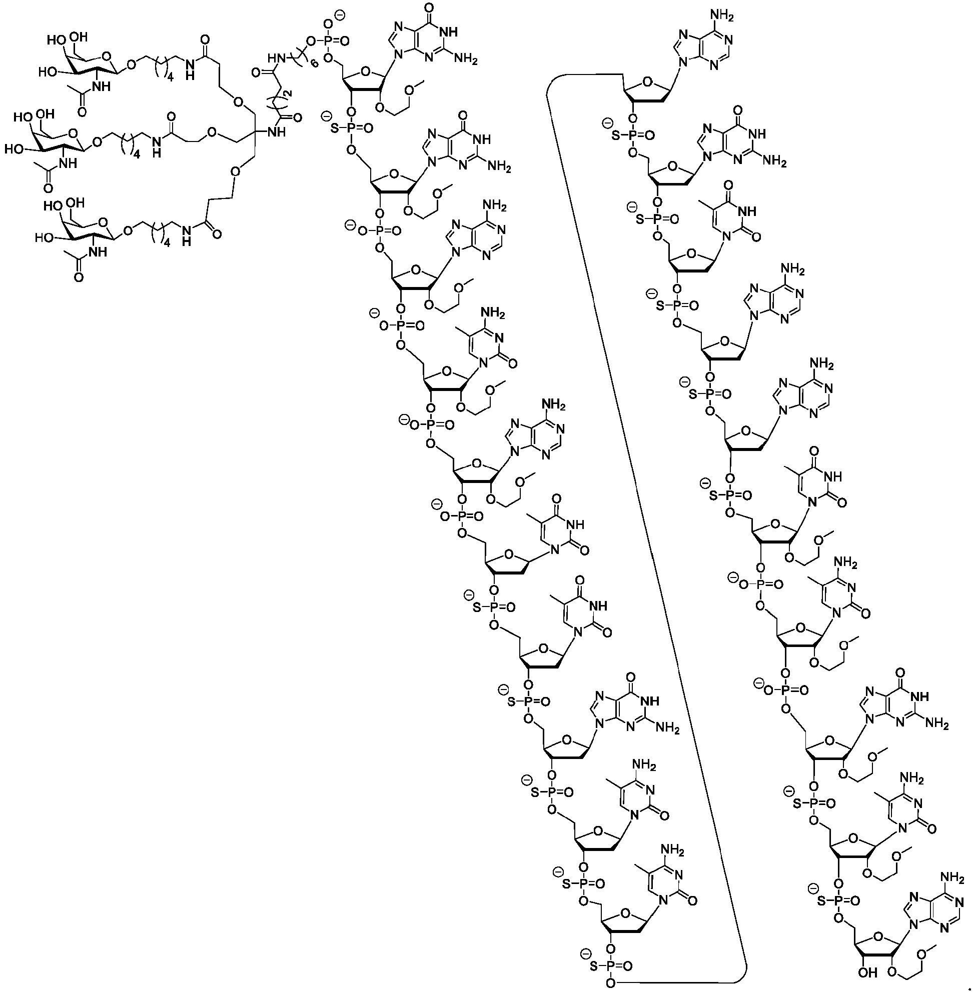

### b. ANGPTL3 Therapeutic Indications

In certain embodiments useful for understanding the invention, the invention provides methods for using a conjugated antisense compound targeted to an ANGPTL3 nucleic acid for modulating the expression of ANGPTL3 in a subject. In certain embodiments useful for understanding the invention, the expression of ANGPTL3 is reduced.

In certain embodiments useful for understanding the invention, the invention provides methods for using a conjugated antisense compound targeted to an ANGPTL3 nucleic acid in a pharmaceutical composition for treating a subject. In certain embodiments useful for understanding the invention, the subject has a metabolic disease and/or cardiovascular disease. In certain embodiments useful for understanding the invention, the subject has combined hyperlipidemia (e.g., familial or non-familial), hypercholesterolemia (e.g., familial homozygous hypercholesterolemia (HoFH), familial heterozygous hypercholesterolemia (HeFH)), dyslipidemia, lipodystrophy, hypertriglyceridemia (e.g., heterozygous LPL deficiency, homozygous LPL deficiency), coronary artery disease (CAD), familial chylomicronemia syndrome (FCS), hyperlipoproteinemia Type IV), metabolic syndrome, non-alcoholic fatty liver disease (NAFLD), nonalcoholic steatohepatitis (NASH), diabetes (e.g., Type 2 diabetes, Type 2 diabetes with dyslipidemia), insulin resistance (e.g., insulin resistance with dyslipidemia), vascular wall thickening, high blood pressure (e.g., pulmonary arterial hypertension), sclerosis (e.g., atherosclerosis, systemic sclerosis, progressive skin sclerosis and proliferative obliterative vasculopathy such as digital ulcers and pulmonary vascular involvement), or a combination thereof.

In certain embodiments useful for understanding the invention, the compounds targeted to ANGPTL3 described herein modulate lipid and/or energy metabolism in a subject. In certain embodiments useful for understanding the invention, the compounds targeted to ANGPTL3 described herein modulate physiological markers or phenotypes of hypercholesterolemia, dyslipidemia, lipodystrophy, hypertriglyceridemia, metabolic syndrome, NAFLD, NASH and/or diabetes. For example, administration of the compounds to a subject can modulate one or more of VLDL, non-esterified fatty acids (NEFA), LDL, cholesterol, triglyceride, glucose, insulin or ANGPTL3 levels. In certain embodiments useful for understanding the invention, the modulation of the physiological markers or phenotypes can be associated with inhibition of ANGPTL3 by the compounds.

In certain embodiments useful for understanding the invention, administration of an antisense compound targeted to an ANGPTL3 nucleic acid results in reduction of ANGPTL3 expression by about at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95% or at least 99%, or a range defined by any two of these values.

### c. Certain ANGPTL3 Dosing Regimens

In certain embodiments useful for understanding the invention, ISIS 703802 is administered to a subject in need thereof. In certain embodiments useful for understanding the invention, 20 mg of ISIS 703802 is administered to a human subject. In certain embodiments useful for understanding the invention, 40 mg of ISIS 703802 is administered to a human subject. In certain embodiments useful for understanding the invention, 80 mg of ISIS 703802 is administered to a human subject. In certain embodiments useful for understanding the invention, 120 mg of ISIS 703802 is administered to a human subject.

In certain embodiments useful for understanding the invention, ISIS 703802 is administered to a subject in need thereof. In certain embodiments useful for understanding the invention, 20 mg of ISIS 703802 is administered to a human subject during a dosing period. In certain embodiments useful for understanding the invention, 40 mg of ISIS 703802 is administered to a human subject during a dosing period. In certain embodiments useful for understanding the invention, 80 mg of ISIS 703802 is administered to a human subject during a dosing period. In certain embodiments useful for understanding the invention, 120 mg of ISIS 703802 is administered to a human subject during a dosing period. In certain embodiments useful for understanding the invention, the dosing period is one week. In certain embodiments useful for understanding the invention, only one dose is given during the dosing period. In certain embodiments useful for understanding the invention, the dosing period is one week.

In certain embodiments useful for understanding the invention, 20 mg of ISIS 703802 is administered to a human subject each week. In certain embodiments useful for understanding the invention, 40 mg of ISIS 703802 is administered to a human subject each week. In certain embodiments useful for understanding the invention, 80 mg of ISIS 703802 is administered to a human subject each week. In certain embodiments useful for understanding the invention, 120 mg of ISIS 703802 is administered to a human subject each week.

### IV. Certain Pharmaceutical Compositions

In certain embodiments, the present invention provides pharmaceutical compositions comprising one or more antisense compound or a salt thereof. In certain such embodiments, the pharmaceutical composition comprises a suitable pharmaceutically acceptable diluent or carrier. In certain embodiments, a pharmaceutical composition comprises a sterile saline solution and one or more antisense compound. In certain embodiments, such pharmaceutical composition consists of a sterile saline solution and one or more antisense compound. In certain embodiments, the sterile saline is pharmaceutical grade saline. In certain embodiments, a pharmaceutical composition comprises one or more antisense compound and sterile water. In certain embodiments, a pharmaceutical composition consists of one antisense compound and sterile water. In certain embodiments, the sterile water is pharmaceutical grade water. In certain embodiments, a pharmaceutical composition comprises one or more antisense compound and phosphate-buffered saline (PBS). In certain embodiments, a pharmaceutical composition consists of one or more antisense compound and sterile PBS. In certain embodiments, the sterile PBS is pharmaceutical grade PBS.

In certain embodiments, pharmaceutical compositions comprise one or more or antisense compound and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, antisense compounds may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

In certain embodiments, pharmaceutical compositions comprising an antisense compound encompass any pharmaceutically acceptable salts of the antisense compound, esters of the antisense compound, or salts of such esters. In certain embodiments, pharmaceutical compositions comprising antisense compounds comprising one or more antisense oligonucleotide, upon administration to an animal, including a human, are capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts. In certain embodiments, prodrugs comprise one or more conjugate group attached to an oligonucleotide, wherein the conjugate group is cleaved by endogenous nucleases within the body.

Lipid moieties have been used in nucleic acid therapies in a variety of methods. In certain such methods, the nucleic acid, such as an antisense compound, is introduced into preformed liposomes or lipoplexes made of mixtures of cationic lipids and neutral lipids. In certain methods, DNA complexes with mono- or poly-cationic lipids are formed without the presence of a neutral lipid. In certain embodiments, a lipid moiety is selected to increase distribution of a pharmaceutical agent to a particular cell or tissue. In certain embodiments, a lipid moiety is selected to increase distribution of a pharmaceutical agent to fat tissue. In certain embodiments, a lipid moiety is selected to increase distribution of a pharmaceutical agent to muscle tissue.

In certain embodiments, pharmaceutical compositions comprise a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, pharmaceutical compositions comprise one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, pharmaceutical compositions comprise a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, pharmaceutical compositions are prepared for oral administration. In certain embodiments, pharmaceutical compositions are prepared for buccal administration. In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may contain formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may contain.

### V. Certain Routes of Administration (application specific)

In certain embodiments, the present disclosure provides methods of administering a pharmaceutical composition comprising an oligonucleotide of the present disclosure to a human. Suitable administration routes include, but are not limited to, oral, rectal, transmucosal, intestinal, enteral, topical, suppository, through inhalation, intrathecal, intracerebroventricular, intraperitoneal, intranasal, intraocular, intratumoral, and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., into the liver).

### Nonlimiting disclosure

While certain compounds, compositions and methods described herein have been described with specificity in accordance with certain embodiments, the following examples serve only to illustrate the compounds described herein and are not intended to limit the same.

Although the sequence listing accompanying this filing identifies each sequence as either "RNA" or "DNA" as required, in reality, those sequences may be modified with any combination of chemical modifications. One of skill in the art will readily appreciate that such designation as "RNA" or "DNA" to describe modified oligonucleotides is, in certain instances, arbitrary. For example, an oligonucleotide comprising a nucleoside comprising a 2'-OH sugar moiety and a thymine base could be described as a DNA having a modified sugar (2'-OH in place of one 2'-H of DNA) or as an RNA having a modified base (thymine (methylated uracil) in place of a uracil of RNA). Accordingly, nucleic acid sequences provided herein, including, but not limited to those in the sequence listing, are intended to encompass nucleic acids containing any combination of natural or modified RNA and/or DNA, including, but not limited to such nucleic acids having modified nucleobases. By way of further example and without limitation, an oligomeric compound having the nucleobase sequence "ATCGATCG" encompasses any oligomeric compounds having such nucleobase sequence, whether modified or unmodified, including, but not limited to, such compounds comprising RNA bases, such as those having sequence "AUCGAUCG" and those having some DNA bases and some RNA bases such as "AUCGATCG" and oligomeric compounds having other modified nucleobases, such as "AT^{m}CGAUCG," wherein ^{m}C indicates a cytosine base comprising a methyl group at the 5-position.

The compounds described herein include variations in which one or more atoms are replaced with a non-radioactive isotope or radioactive isotope of the indicated element. For example, compounds herein that comprise hydrogen atoms encompass all possible deuterium substitutions for each of the ¹H hydrogen atoms. Isotopic substitutions encompassed by the compounds herein include but are not limited to: ²H or ³H in place of ¹H, ¹³C or ¹⁴C in place of ¹²C, ¹⁵N in place of ¹⁴N, ¹⁷O or ¹⁸O in place of ¹⁶O, and ³³S, ³⁴S, ³⁵S, or ³⁶S in place of ³²S. In certain embodiments, non-radioactive isotopic substitutions may impart new properties on the oligomeric compound that are beneficial for use as a therapeutic or research tool. In certain embodiments, radioactive isotopic substitutions may make the compound suitable for research or diagnostic purposes such as imaging.

### EXAMPLES

The following examples illustrate certain embodiments of the present disclosure and are not limiting. Moreover, where specific embodiments are provided, the inventors have contemplated generic application of those specific embodiments. For example, disclosure of an oligonucleotide having a particular motif provides reasonable support for additional oligonucleotides having the same or similar motif. And, for example, where a particular high-affinity modification appears at a particular position, other high-affinity modifications at the same position are considered suitable, unless otherwise indicated.

### Reference Example 1: ISIS 681257 Clinical Trial

As described herein, a double-blinded, placebo-controlled, dose-escalation Phase 1 study was performed on healthy volunteers with elevated Lp(a) to assess safety, tolerability, pharmacokinetics (PK) and pharmacodynamics (PD) after administration of single and multiple doses of ISIS 681257. ISIS 681257 was previously disclosed in WO 2014/179625 and is also described hereinabove. ISIS 681257 has been shown to be potent in inhibiting Lp(a) and tolerable when administered to non-human subjects. This subsequent study revealed unexpectedly improved properties of ISIS 681257 when administered to human subjects.

### Screening

Up to 28 days prior to treatment, subjects were screened for eligibility to participate in the study. Admission criteria for the study include the following:
1. Healthy males or females aged 18-65 inclusive and weighing ≥ 50 kg at the time of informed consent
2. BMI < 35.0 kg/m2
3. Subjects must have Lp(a) ≥ 75 nanomoles/liter (nmol/L) (≥ 30 mg/dL) at Screening. The Lp(a) value obtained via the Lp(a) pre-screening protocol may also be used to meet this criterion if measured within 6 months of dosing.

### Study Drug

Solutions of the Study Drug ISIS 681257 (100 mg/mL, 0.8 mL) contained in stoppered glass vials was used. Vials were for single use only. Doses of ISIS 681257 solution and placebo (0.9% sterile saline) were prepared by an unblinded pharmacist (or qualified delegate). A trained professional administered the ISIS 681257 or placebo blindly as a subcutaneous (sc) injection(s) in the abdomen, thigh, or outer area of the upper arm on each dosing day.

### Treatment and Post-Treatment Evaluation

Subjects enrolled in the study were split into 2 treatment arms: Single Ascending Dose (SAD) or Multiple Ascending Dose (MAD).

### Example 1A: Single Ascending Dose (SAD)

Approximately 28 subjects were enrolled in the SAD arm of this study, grouped into cohorts of 4 or 8 subjects randomized 3:1, ISIS 681257 to placebo. The subjects were administered placebo or ISIS 681257 at the doses listed in Table 1.

**Table 1: Single Ascending Doses**

| Cohort (n) | Dose |
|---|---|
| A (4) | 10 mg |
| B (4) | 20 mg |
| C (4) | 40 mg |
| D (8) | 80 mg |
| E (8) | 120 mg |

After treatment with a single dose of ISIS 681257 or placebo, the subjects were followed for up to 90 days to monitor the safety, tolerability, PK and PD of the drug. During the follow-up period, subjects return to the Study Center for visits on Study Days 2, 3, 8, 15 and 30 post-treatment (and Days 50, 70 and 90 post-treatment for Cohorts C, D and E) for safety and laboratory evaluations (blood draws), monitoring, concomitant medication usage recording, and adverse event (AE) collection. Collection of urine and feces was also performed on certain days. All visits by the subject for post-treatment assessment had a visit window of up to ± 1 days.

Analysis of serum samples showed dose dependent reductions in Lp(a) levels after a single dose of ISIS 681257 as measured 2 days, 4 days, 8 days, 15 days and 30 days post-treatment (Cohorts C, D and E were also assessed about 50 days, 70 days and 90 days post-treatment). Results, presented as a mean percent change in Lp(a) from baseline, are shown in Table 2.

**Table 2: Dose-dependent Change in Lp(a) after a Single Dose of ISIS 681257**

| | % Change from Baseline | | | | | | | |
|---|---|---|---|---|---|---|---|---|
| Cohort | Day 2 | Day 4 | Day 8 | Day 15 | Day 30 | Day 50 | Day 70 | Day 90 |
| Placebo | -1.7 | -2.4 | -9.6 | 0.3 | 6.8 | -14 | -9 | 3.9 |
| A | 4 | -5 | -16 | -20 | -26 | - | - | - |
| B | 7 | 8 | 2 | -22 | -33 | - | - | - |
| C | -2 | -13 | -33 | -41 | -43 | -35 | -26 | -26 |
| D | -21 | -35 | -50 | -70 | -79 | -71 | -52 | -46 |
| E | -11 | -25 | -50 | -76 | -85 | -75 | -61 | -44 |

Additionally, analysis of apo(a) isoforms, lipoprotein-associated phospholipase A2 (Lp-PLA2), secretory phospholipase A2 (sPLA2), oxidized phospholipid associated with apolipoprotein B (OxPL-apoB), and oxidized phospholipid associated with apolipoprotein(a) (OxPL-apo(a)) were performed.

During scheduled visits to the Study Center, the safety and tolerability of ISIS 681257 was clinically assessed in the subjects. Clinical staff assessed safety and tolerability by collecting and/or measuring one or more of the following: adverse events (AEs), quality of life assessments, concomitant medication/procedure information, vital signs, physical examination results (e.g., injection site reactions (ISRs) or flu-like symptoms (FLSs)), waist circumference, skinfold measurements, DEXA scans, electrocardiograms (ECGs), liver MRIs and echocardiograms.

Laboratory measurements such as serum chemistry (e.g., ALT, AST, bilirubin, creatinine, BUN), urinalysis, coagulation (e.g., aPTT (sec), PT (sec), INR, plasminogen), complement (e.g., C5a, Bb), hematology (e.g., hematocrit, white blood cells, platelets), immune function, thyroid function, inflammation (hsCRP), lipid panel (e.g., total cholesterol, HDL, LDL, TG, apoB, VLDL), ISIS 681257 plasma trough concentrations, and/or immunogenicity testing were performed on subject samples to assess the health and safety of each subject and the PD of the drug.

Laboratory measurements of subject samples were also used for PK profiling of the drug. For example, samples were used for measuring the amount and stability of ISIS 681257 and/or metabolites thereof, assessing drug binding proteins, and/or assessing other actions of ISIS 681257 with plasma constituents.

Both single dose treatment and multiple dose treatment with ISIS 681257 did not result in any safety or tolerability issues, at any of the clinically revelant doses tested. No ISRs were observed and no side effects were noted in any laboratory tests and the liver enzymes ALT and AST were not elevated.

The above results were surprising, because earlier experiments involving both the unconjugated compound (ISIS 494372) and the GalNAc conjugated compound (ISIS 681257) had suggested that the GalNAc conjugated compound would have significantly lower potency and/or a shorter duration of action in humans than was observed following the first dosing of humans reported herein (*e.g.* see Examples 89, 100 and 108 of WO 2014/179625 and Tsimikas et al., Lancet, 2015 Oct 10; 386:1472-83). In light of these surprising results, when treating humans, the GalNAc conjugated compound (ISIS 681257, or a salt thereof) can be administered at lower doses and/or less frequently than expected based on the earlier *in vivo* testing of the GalNAc conjugated compound. This can provide one or more very significant improvements in treating humans, *e.g*. reduced cost of treatment, improved patient compliance, reduced volume of administered medicinal product and/or potentially reduced risk of potential adverse events via lower dose administration regimens.

### Example 1B: Multiple Ascending Dose (MAD)

Thirty subjects were enrolled in the MAD arm of this study, grouped into cohorts of 10 randomized 4:1, ISIS 681257 to placebo. The subjects were administered a placebo or ISIS 681257 at the doses listed in Table 3. A total of 6 doses of drug or placebo was administered to each subject: loading doses were administered in the first week on Study Days 1 (first dose), 3, 5 and 8; maintenance doses were then administered weekly on Study Days 15 and 22.

**Table 3: Multiple Ascending Doses**

| Cohort (n) | ISIS 681257 Dose | # of Doses | Total Drug Dose |
|---|---|---|---|
| AA (10) | 10 mg | 6 | 60 mg |
| BB (10) | 20 mg | 6 | 120 mg |
| CC (10) | 40 mg | 6 | 240 mg |

During treatment with ISIS 681257 or placebo and for up to 13 weeks after treatment, the subjects were monitored for safety, tolerability, PK and PD of the drug. During the treatment period and the follow-up period, subjects return to the Study Center for visits on Study Days 5, 8, 15, 22, 29, 36, 50, 64, 85 and 113 for safety and clinical laboratory evaluations (blood draws), monitoring, concomitant medication usage recording, and AE event collection. Collection of urine and feces was also performed on certain days. All visits by the subject for post-treatment assessment had a visit window of up to ± 1 days.

Analysis of serum samples showed reductions in Lp(a) levels after multiple doses of ISIS 681257 as measured 5 days, 8 days, 15 days, 22 days, 29 days and 36 days after start of treatment. Results, presented as a mean percent change in Lp(a) from baseline, are shown in Table 4. Surprisingly, after a single dose of ISIS 681257, levels of Lp(a) continue to fall, reaching a nadir at about day 50 for the AA cohort. This demonstrates that the effective half life of ISIS 681257 appears to be much longer than anticipated. Additionally, cohorts BB and CC demonstrate continued reduction in Lp(a) through 36 days after administration of ISIS 681257.

**Table 4: Dose-dependent Reductions in Lp(a) after a Multiple Doses of ISIS-681257**

| | % Change from Baseline | | | | | | | | | |
|---|---|---|---|---|---|---|---|---|---|---|
| Cohort | Day 5 | Day 8 | Day 15 | Day 22 | Day 29 | Day 36 | Day 50 | Day 64 | Day 85 | Day 113 |
| Placebo | -2.6 | -11 | -11 | -4 | -3 | -10 | -1 | 18 | -1 | 10 |
| AA | -9 | -23 | -43 | -50 | -61 | -72 | -68 | -66 | -52 | -39 |
| BB | -10 | -20 | -52 | -68 | -75 | -80 | -80 | -77 | -64 | -52 |
| CC | -19 | -44 | -71 | -84 | -90 | -94 | -90 | -85 | -73 | -58 |

**Table 5: ED50 Values in Human**

| Regimen | ISIS 494372 | ISIS 681257 |
|---|---|---|
| Weekly Dose ED50 | 145 mg (1.5 ml) | 4.5 mg (0.05 ml) |

In human subjects, ISIS 681257 displayed dose-dependent, durable, statistically significant reductions in Lp(a) and an ED50 of 4.5 mg. ISIS 681257 was unexpectedly found to be ≥ 30-fold more potent than ISIS 494372 (an unconjugated antisense compound of the same nucleobase sequence and length; previously described in WO 2013/177468). Earlier experiments involving both ISIS 494372 and ISIS 681257 (reported in WO 2014/179625) had indicated that the GalNAc conjugated compound benefits from higher *in vivo* potency in mice, but these earlier experiments did not reveal or predict the unexpected ≥ 30-fold improvement in humans. Additionally, for the 10 mg multi-dose cohort, data points past Day 36 indicate that the nadir of Lp(a) levels for 6 of the 8 patients was not achieved until about Day 50, indicating that in humans ISIS 681257 showed an unexpected long half-life (T1/2) compared to ISIS 494372 (Tsimikas et al., Lancet, 2015 Oct 10; 386:1472-83) and this was likewise not revealed or predicted by earlier experiments in mice involving both ISIS 494372 and ISIS 681257.

Additionally, analysis of apo(a) isoforms, lipoprotein-associated phospholipase A2 (Lp-PLA2), secretory phospholipase A2 (sPLA2), oxidized phospholipid associated with apolipoprotein B (OxPL-apoB), and oxidized phospholipid associated with apolipoprotein(a) (OxPL-apo(a)) were performed. The results showed a significant reduction in LDL cholesterol, apolipoprotein B (apoB), and oxidised phospholipids (OxPL) associated with apoB and apo(a). Therefore, the reductions in Lp(a) occurred alongside significant reductions in proinflammatory OxPL, as well as reductions in LDL-C and apoB-100, which is consistent with a salutary effect on several causal pathways that mediate cardiovascular disease and calcific aortic valve stenosis. *See* Viney, et al. Lancet, 2016, Sep 2016; 388: 2239-53.

During scheduled visits to the Study Center, the safety and tolerability of ISIS 681257 was clinically assessed in the subjects. Clinical staff assessed safety and tolerability by collecting and/or measuring one or more of the following: adverse events (AEs), quality of life assessments, concomitant medication/procedure information, vital signs, physical examination results (e.g., injection site reactions (ISRs) or flu-like symptoms (FLSs)), waist circumference, skinfold measurements, DEXA scans, electrocardiograms (ECGs), liver MRIs and echocardiograms.

Laboratory measurements such as serum chemistry (e.g., ALT, AST, bilirubin, creatinine, BUN), urinalysis, coagulation (e.g., aPTT (sec), PT (sec), INR, plasminogen), complement (e.g., C5a, Bb), hematology (e.g., hematocrit, white blood cells, platelets), immune function, thyroid function, inflammation (hsCRP), lipid panel (e.g., total cholesterol, HDL, LDL, TG, apoB, VLDL), ISIS 681257 plasma trough concentrations, and/or immunogenicity testing were performed on subject samples to assess the health and safety of each subject and the PD of the drug.

Laboratory measurements of subject samples were also used for PK profiling of the drug. For example, samples were used for measuring the amount and stability of ISIS 681257 and/or metabolites thereof, assessing drug binding proteins, and/or assessing other actions of ISIS 681257 with plasma constituents.

Multiple dose treatments with ISIS 681257 did not result in any safety or tolerability issues. No ISR or FLS were observed. Liver enzymes ALT and AST were not elevated.

The ≥ 30-fold improvement in potency in humans was significantly greater than that expected. The above results were surprising, because earlier experiments involving both the unconjugated compound (ISIS 494372) and the GalNAc conjugated compound (ISIS 681257) had suggested that the GalNAc conjugated compound would have significantly lower potency and/or a shorter duration of action in humans than was observed following the first dosing of humans reported herein (*e.g.* see Examples 89, 100 and 108 of WO 2014/179625 and Tsimikas et al., Lancet, 2015 Oct 10; 386:1472-83). In light of these surprising results, when treating humans, the GalNAc conjugated compound (ISIS 681257, or a salt thereof) can be administered at lower doses and/or less frequently than expected based on the earlier *in vivo* testing of the GalNAc conjugated compound. This can provide one or more very significant improvements in treating humans, e.g. reduced cost of treatment, improved patient compliance, reduced volume of administered medicinal product and/or potentially reduced risk of potential adverse events via lower dose administration regimens.

### Reference Example 2: Dose Regimens

Modeling based on the Phase 1clinical trial results was performed to assess optimal clinical dose regimens for ISIS 681257.

### Weekly Dosing

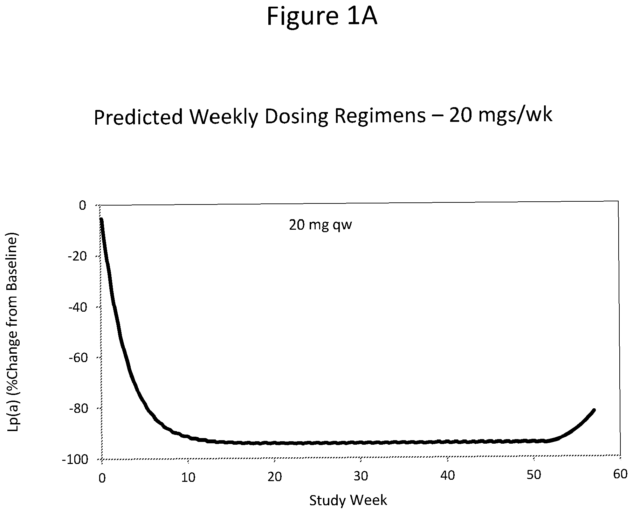

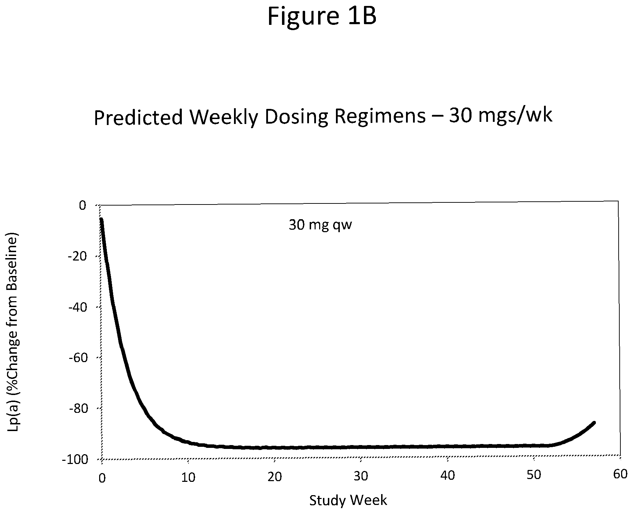

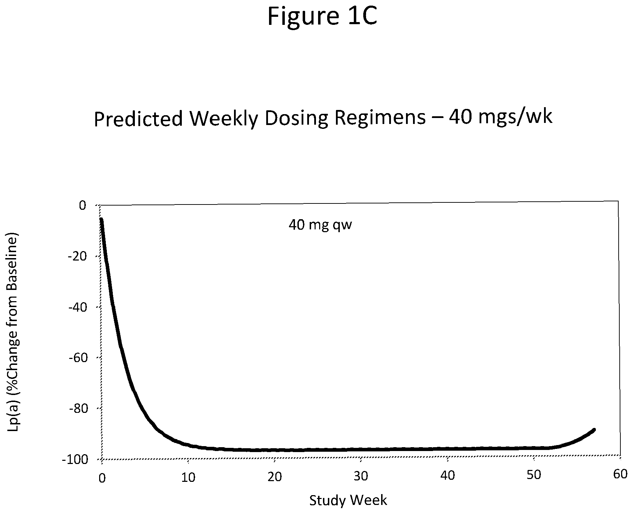

Figures 1A-C. Predicted Weekly Dosing Regimens. Charts are shown modeling the effect on Lp(a) by weekly administration of ISIS 681257 at doses of 20 mg (Fig 1A), 30 mg (Fig 1B) or 40 mg (Fig 1C). Lp(a) shows a steady state reduction of ≥ 80%.

### Monthly Dosing

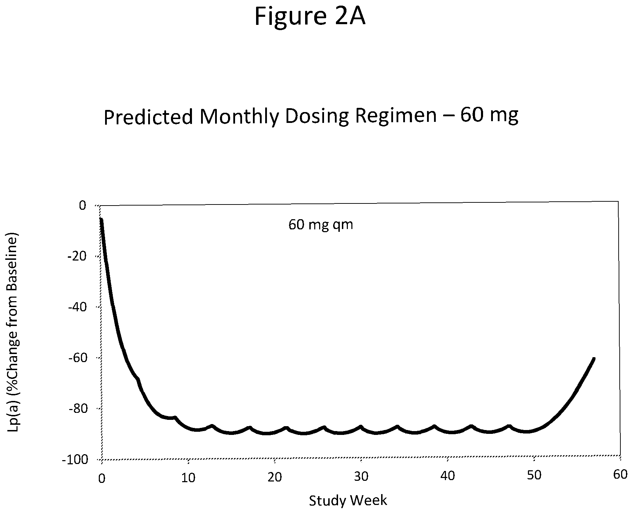

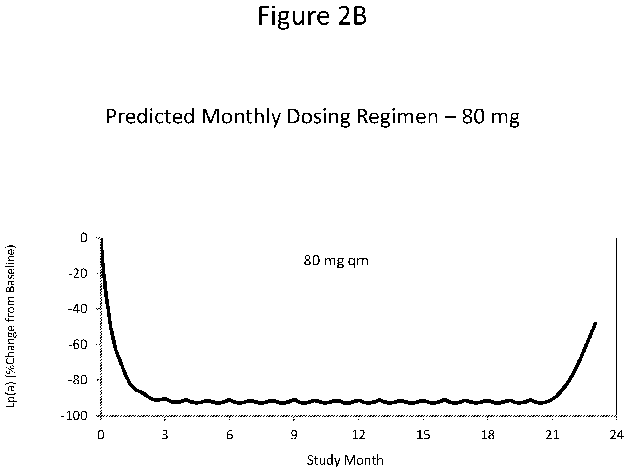

Figures 2A-B. Predicted Monthly Dosing Regimens. Chart are shown modeling the effect on Lp(a) by monthly administration of ISIS 681257 at dose of 60 mg (Fig 2A) and 80 mg (Fig 2B). Lp(a) shows a steady state reduction of about 80%.

### Two Months Dosing

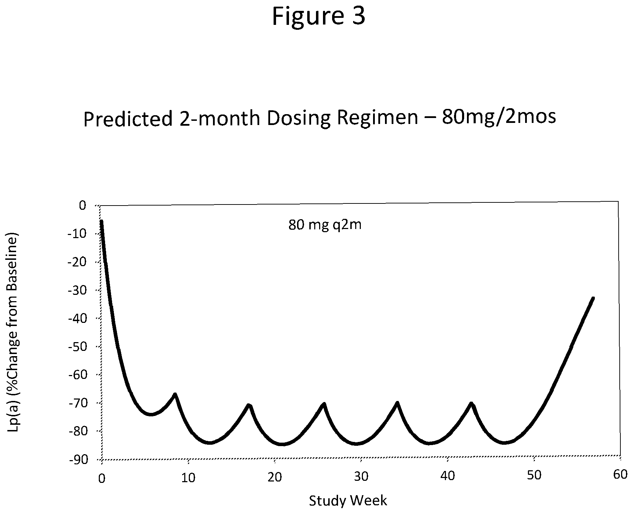

Figure 3. Predicted 2-month Dosing Regimen. A chart is shown modeling the effect on Lp(a) by administration of ISIS 681257 at an 80 mg dose every 2-months. Lp(a) shows a steady state reduction of about 80%.

### Quarterly dosing

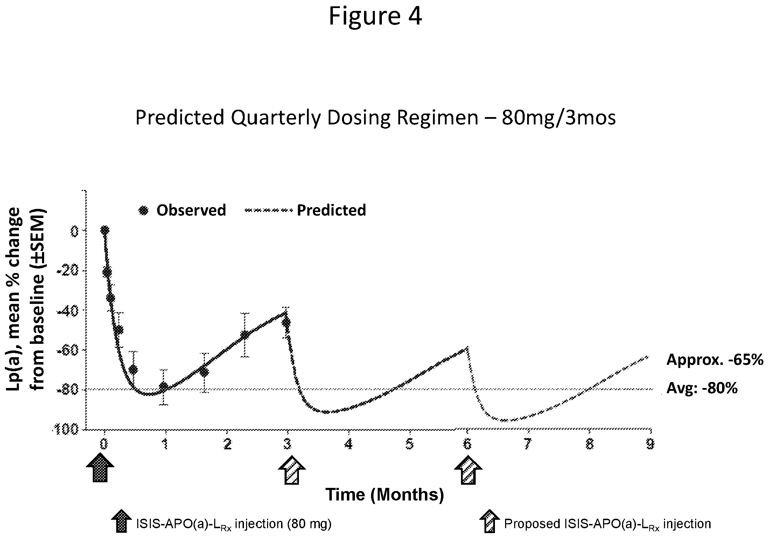

Figure 4. Predicted Quarterly Dosing Regimen. A chart is shown modeling the effect on Lp(a) by quarterly administration of ISIS 681257 at an 80 mg dose. Lp(a) shows a steady state reduction of 80% and maximum reduction of > 90%.

### Reference Example 3: Dose Regimens

After completion of the phase 1 study described above, further modeling was performed to assess optimal clinical dose regimens for ISIS 681257.

### Weekly Dosing

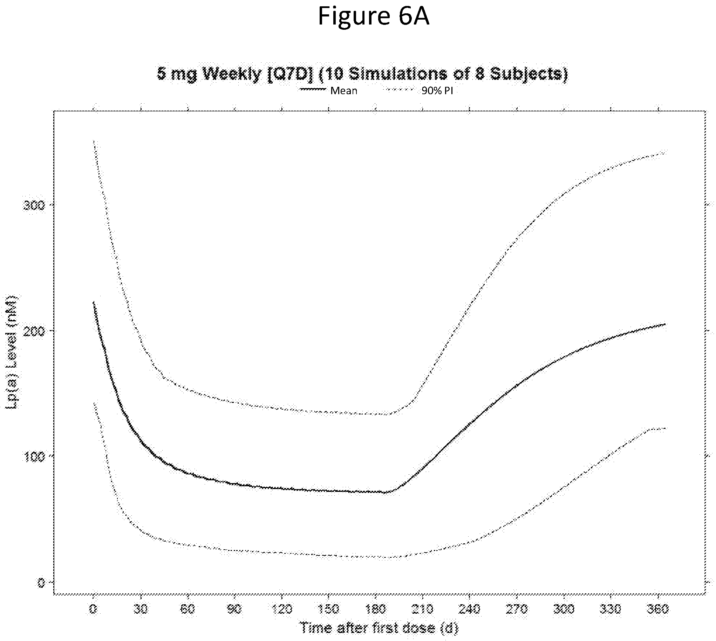

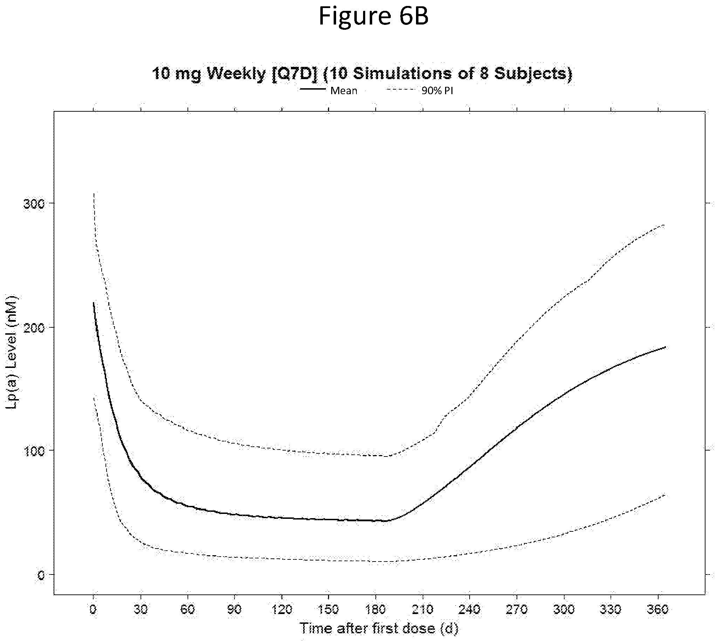

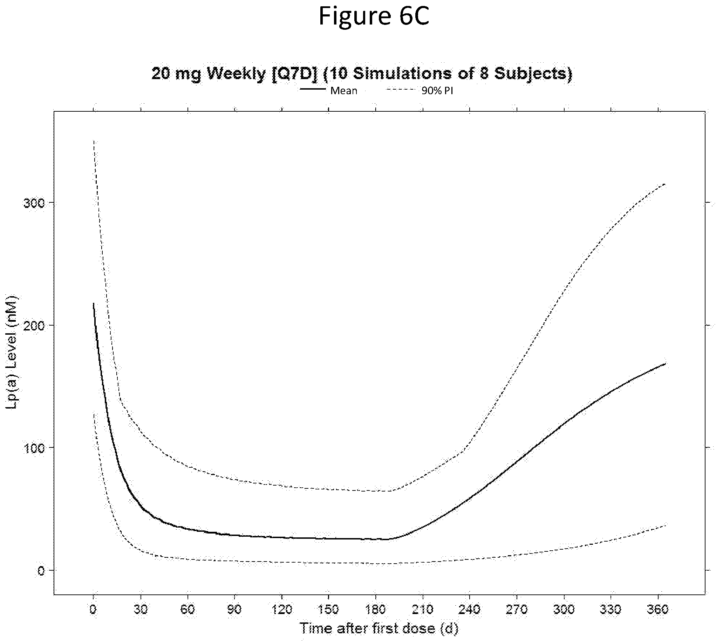

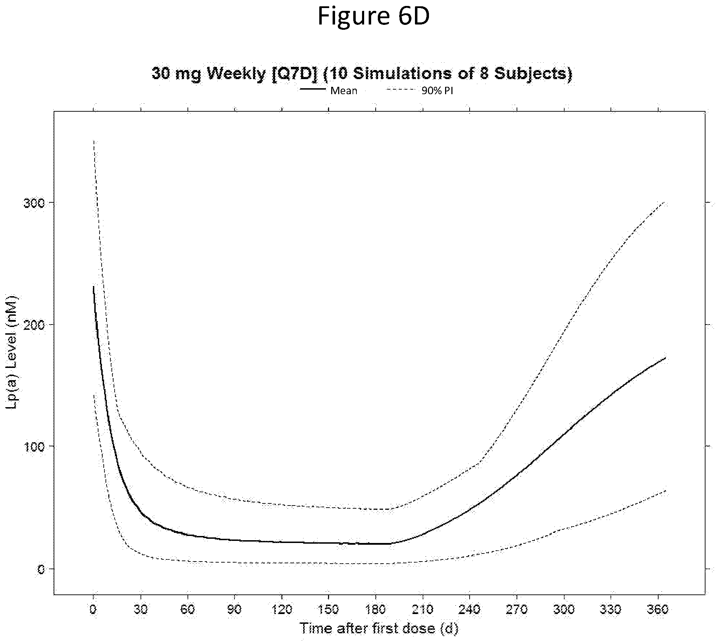

Figures 6A-D. Predicted Weekly Dosing Regimens. Charts are shown modeling the effect on Lp(a) by weekly administration of ISIS 681257 at doses of 5 mg (Fig 6A), 10 mg (Fig. 6B), 20 mg (Fig. 6C), and 30 mg (Fig. 6D). The dark middle line represents the predicted dose, while the uppermost and lowermost lines represent the 90% Confidence Interval.

### Monthly Dosing

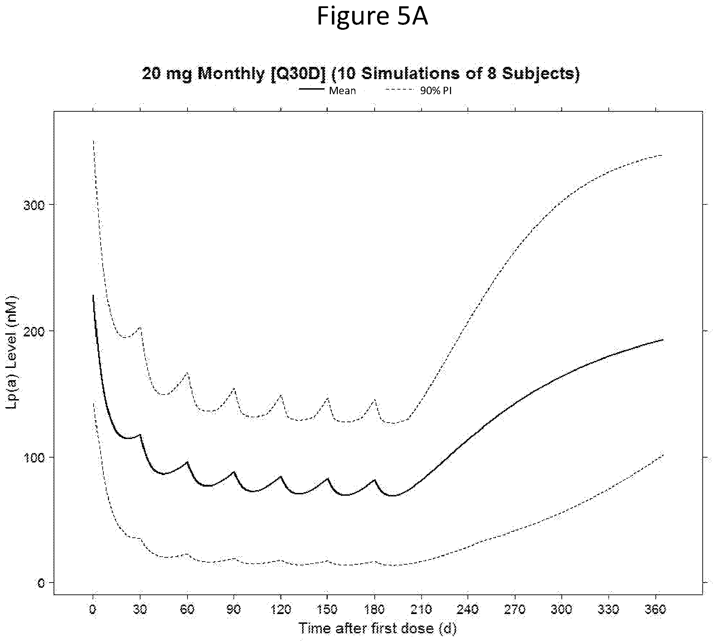

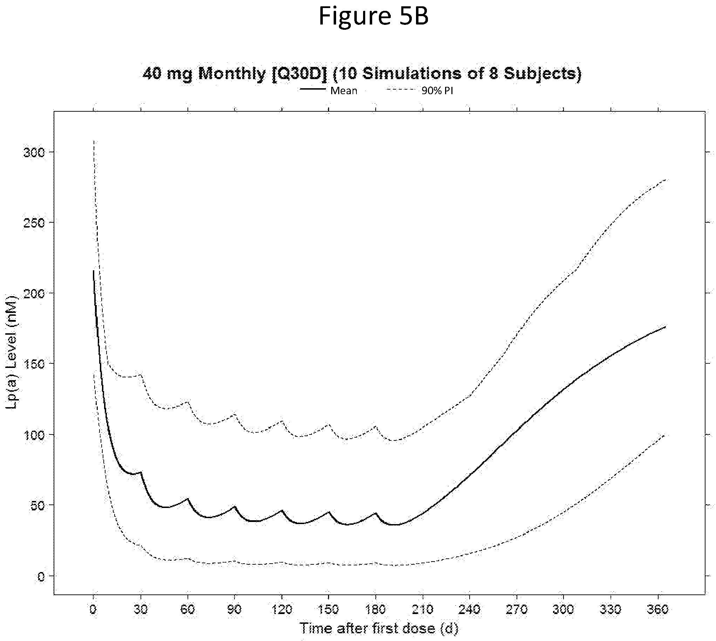

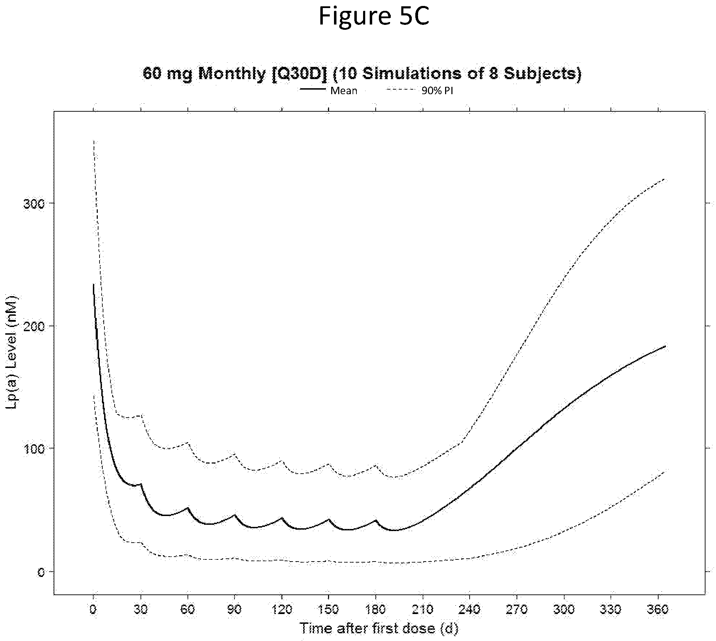

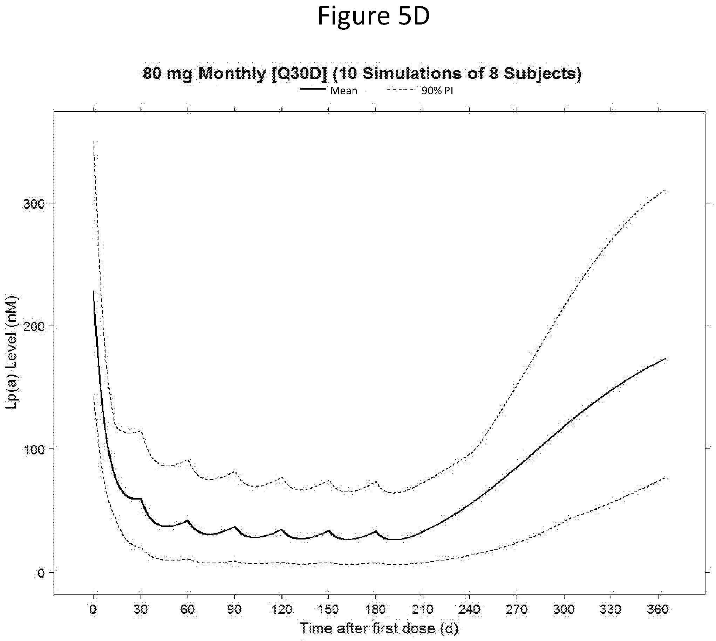

Figures 5A-D. Predicted Monthly Dosing Regimens. Charts are shown modeling the effect on Lp(a) by monthly administration of ISIS 681257 at doses of 20 mg (Fig 5A), 40 mg (Fig. 5B), 60 mg (Fig. 5C), and 80 mg (Fig.5D ). The dark middle line represents the predicted dose, while the uppermost and lowermost lines represent the 90% Confidence Interval.

### Reference

### Example 4: A Randomized, Double-blind, Placebo-Controlled, Dose-Ranging Phase 2 Study of ISIS 681257 Administered Subcutaneously to Patients with Hyperlipoproteinemia(a) and Established Cardiovascular Disease (CVD)

The study described herein is to evaluate the safety, including tolerability, of ISIS 681257 and to assess the efficacy of different doses and dosing regimens of ISIS 681257 for reduction of plasma Lp(a) levels in patients with hyperlipoproteinemia(a) and established cardiovascular disease (CVD). CVD is defined as documented coronary artery disease, stroke, or peripheral artery disease. Patients must also have Lp(a) plasma level of ≥ 60 mg/dL. ISIS 681257 may provide therapeutic benefits to patients that have hyperlipoproteinemia(a) and established CVD.

Patient doses may be either 10 mg or 20 mg of ISIS 681257 administered once per week via subcutaneous injection for 52 weeks. Additional patient doses may be either 20mg, 40mg, or 60mg administered once every 4 weeks via subcutaneous injection for up to 13 administrations. The primary endpoint is the percent change in plasma Lp(a) from baseline at the primary analysis time point for ISIS 681257 treatment groups compared to placebo. The primary analysis time point is at Week 25 for patients who received every 4-week dosing and at Week 27 for patients who received weekly dosing. Secondary empoints may comprise the effect of ISIS 681257 as compared to placebo at the primary analysis time point on any one of the following:
- Percent change from baseline in LDL-C;
- Proportion of patients who achieve plasma Lp(a) ≤ 50 mg/dL;
- Proportion of patients who achieve plasma Lp(a) < 30 mg/dL;
- Percent change from baseline in apoB;
- Percent change from baseline in OxPL-apo(a); and/or
- Percent change from baseline in OxPL-apoB.
This study may reveal unexpectedly improved properties of ISIS 681257 when administered to human subjects with hyperlipoproteinemia(a) and established cardiovascular disease (CVD). Treatment with ISIS 681257 may produce reduction in Lp(a) in patients with hyperlipoproteinemia(a) and established cardiovascular disease (CVD).

### Example 5: ISIS 678354 Clinical Trial

The study described herein is to evaluate safety and tolerability of single and multiple doses (both weekly and every 4 weeks dosing regimens) of ISIS 678354 administered subcutaneously (SC) to healthy subjects with elevated triglycerides (TG). ISIS 678354 may provide therapeutic benefits to patients that have elevated triglycerides.

The study described herein has both a single ascending dose arm and a multiple ascending dose arm. In the single dose arm, patient doses may be either 20 mg, 40 mg, 80mg, or 120mg of ISIS 678354 administered via subcutaneous injection. In the multiple dose arm, patient doses may be either 20mg, 40mg, or 80mg administered once every week via subcutaneous injection for up to 6 administrations. The pharmacodynamics of ISIS 678354 will then be meausured for each patient to assess: Absolute and percent change from baseline in fasting TG, apoC-III, LDL-C, HDL-C, VLDL-C, TC, non-HDL-C, apolipoprotein A-1 (apoA-I), apoB, LDL:HDL ratio, TC:HDL ratio, and lipoprotein (a) (Lp(a)). As used herein, "LDL-C" means low-density lipoprotein cholesterol. As used herein, "HDL-C" means high-density lipoprotein cholesterol. As used herein, "VLDL-C" means very low-density lipoprotein cholesterol.

This study may reveal unexpectedly improved properties of ISIS 678354 when administered to human subjects with elevated triglycerides. Treatment with ISIS 678354 may produce reduction in triglycerides. Treatment with ISIS 678354 may produce reduction in LDL-C. Treatment with ISIS 678354 may produce reduction in VLDL-C. Treatment with ISIS 678354 may produce increase in HDL-C.

### Reference Example 6: ISIS 703802 Clinical Trial

The study described herein is to evaluate the safety and tolerability of single and multiple doses of ISIS 703802 administered subcutaneously (SC) to healthy subjects with elevated triglycerides (TG) and subjects with familial hypercholesterolemia. ISIS 703802 may provide therapeutic benefits to patients that have elevated triglycerides and/or familial hypercholesterolemia.

The study described herein has both a single ascending dose arm and a multiple ascending dose arm. In the single dose arm, patient doses may be either 20 mg, 40 mg, 80mg, or 120mg of ISIS 703802 administered via subcutaneous injection. In the multiple dose arm, patient doses may be either 20mg, 40mg, 80mg, or 120mg administered once every week via subcutaneous injection for up to 6 administrations. The pharmacodynamics of ISIS 703802 will then be meausured for each patient to assess plasma angiopoietin-like 3 (ANGPTL3), total cholesterol (TC), low density lipoprotein cholesterol (LDL-C), high density lipoprotein cholesterol (HDL-C), non-high density lipoprotein cholesterol (non-HDL-C), very low density lipoprotein cholesterol (VLDL-C), and TG.

This study may reveal unexpectedly improved properties of ISIS 703802 when administered to human subjects with elevated triglycerides. Treatment with ISIS 703802 may produce reduction in triglycerides. Treatment with ISIS 703802 may produce reduction in LDL-C. Treatment with ISIS 703802 may produce reduction or amelioration of one or more symptoms associated with familial hypercholesterolemia.

## Claims

1. An oligomeric compound for use in treating or preventing a disease or condition in a human, wherein the treatment comprises administering not more than 300mg of the oligomeric compound to the human during a dosing period of four weeks, wherein the anion form of the oligomeric compound has the following chemical structure:

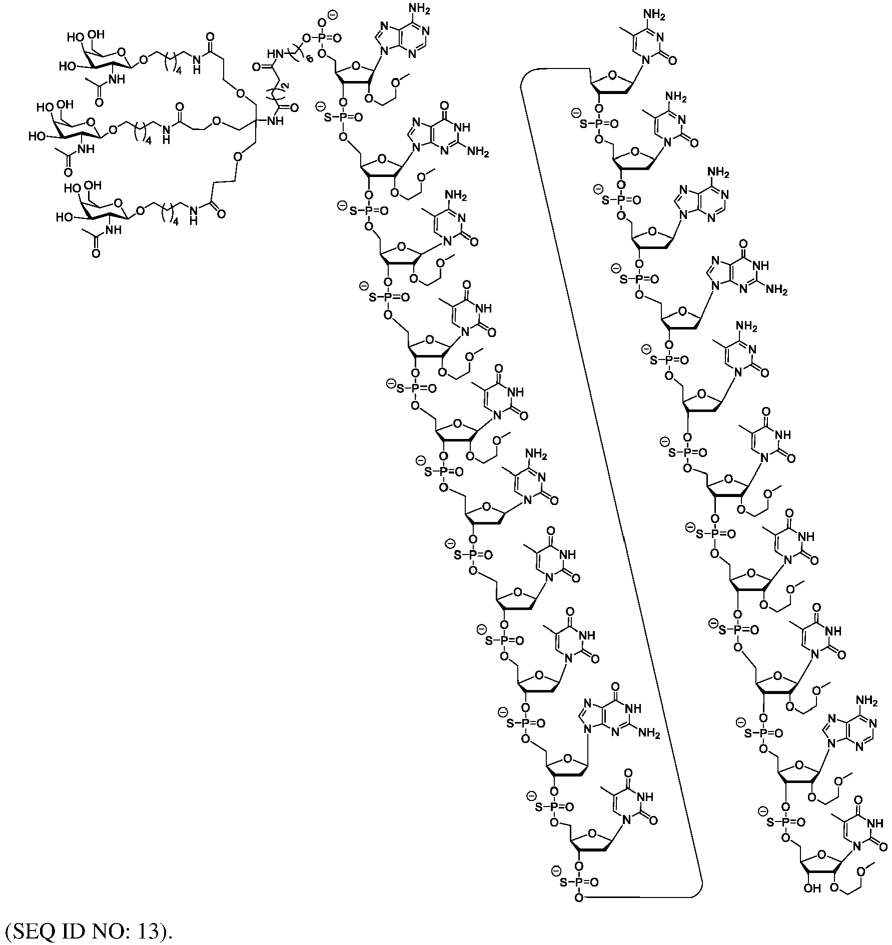

2. The oligomeric compound for use according to claim 1, wherein the oligomeric compound is a salt, and wherein the cation of the salt is sodium or potassium.

3. The oligomeric compound for use according to any preceding claim, wherein the treatment comprises administering not more than 250mg, not more than 240mg, not more than 120mg, not more than 100mg, not more than 80mg, not more than 60mg, not more than 40mg, not more than 30mg, not more than 20mg, not more than 10mg, or not more than 5mg of the oligomeric compound to the human during the dosing period.

4. The oligomeric compound for use according to any preceding claim, wherein:
(i) the treatment comprises administering a unit dose comprising not more than 250mg, not more than 120mg, not more than 100mg, not more than 80mg, not more than 75mg, not more than 60mg, not more than 50mg, not more than 40mg, not more than 30mg, not more than 25mg, not more than 20mg, not more than 15mg, not more than 10mg, or not more than 5mg of the oligomeric compound, or
(ii) the treatment comprises administering a unit dose:
(A) of from 115mg to 125mg, optionally 120 mg, of the oligomeric compound,
(B) of from 95mg to 105mg, optionally 100 mg, of the oligomeric compound,
(C) of from 75mg to 85mg, optionally 80 mg, of the oligomeric compound,
(D) of from 55mg to 65mg, optionally 60 mg, of the oligomeric compound,
(E) of from 45mg to 55mg, optionally 50 mg, of the oligomeric compound,
(F) of from 35mg to 45mg, optionally 40 mg, of the oligomeric compound,
(G) of from 25mg to 35mg, optionally 30 mg, of the oligomeric compound,
(H) of from 15mg to 25mg, optionally 20 mg, of the oligomeric compound,
(I) of from 5mg to 15mg, optionally 10 mg, of the oligomeric compound, or
(J) of from 1mg to 10mg, optionally 5 mg, of the oligomeric compound, or
(iii) the treatment comprises administering a unit dose comprising not less than 1mg, not less than 2.5mg, or not less than 5mg of the oligomeric compound, or
(iv) the treatment comprises administering a unit dose comprising:
(A) 20 mg of the oligomeric compound,
(B) 40 mg of the oligomeric compound,
(C) 80 mg of the oligomeric compound, or
(D) 120 mg of the oligomeric compound.

5. The oligomeric compound for use according to any preceding claim, wherein the treatment comprises administering not more than 1 unit dose, not more than 2 unit doses, not more than 3 unit doses, not more than 4 unit doses, not more than 5 unit doses, or not more than 6 unit doses to the human during the dosing period.

6. The oligomeric compound for use according to any of claims 1-4, wherein the treatment dosing regimen comprises administering a unit dose once every week, every two weeks, every three weeks, every four weeks, every month, every two months, or every three months.

7. The oligomeric compound for use according to any preceding claim, wherein the oligomeric compound is administered by subcutaneous injection, optionally by subcutaneous injection into the abdomen, thigh, or upper arm.

8. The oligomeric compound for use according to any preceding claim, wherein the oligomeric compound is administered by injection, optionally wherein:
(a) the oligomeric compound is formulated in a sterile liquid and optionally wherein each unit dose of the oligomeric compound is not more than 1 mL, not more than 0.8 mL, not more than 0.5 mL, or not more than 0.25 mL of the sterile liquid,
optionally wherein the sterile liquid is selected from among: saline and water, optionally wherein the sterile liquid further comprises a buffer and/or sodium chloride, and/or
(b) the oligomeric compound is formulated as a sodium salt.

9. The oligomeric compound for use according to any preceding claim, wherein the treatment reduces the fasting plasma triglyceride concentration in the human by at least 30%, when the fasting plasma triglyceride concentration in the human is measured at the start and end of the dosing period.

10. The oligomeric compound for use according to any preceding claim, wherein the treatment comprises administration to a subject having one or more symptoms of a cardiovascular disease or disorder, and wherein the treatment ameliorates one or more symptoms of the cardiovascular disease or disorder.

11. The oligomeric compound for use according to any of claims 1-9, wherein the treatment comprises administration to a subject having cardiovascular disease, metabolic syndrome, obesity and/or diabetes.

12. The oligomeric compound for use according to any of claims 1-9, wherein the treatment comprises administration to a subject having hypertriglyceridemia, mixed dyslipidemia, atherosclerosis, a risk of developing atherosclerosis, coronary heart disease, one or more risk factors for coronary heart disease, diabetes, hepatic steatosis, and/or pancreatitis.

13. The oligomeric compound for use according claim 12, wherein the treatment comprises administration to a subject having Type II diabetes and/or hypertriglyceridemia.

14. The oligomeric compound for use according to claim 12, wherein the treatment comprises administration to a subject having familial hypertriglyceridemia, non-familial hypertriglyceridemia, heterozygous familial hypertriglyceridemia, and/or homozygous familial hypertriglyceridemia.

15. A pharmaceutical composition, comprising:
(i) an oligomeric compound, wherein the oligomeric compound is an oligomeric compound as defined in claim 1 or claim 2, and
(ii) one or more pharmaceutically acceptable carriers or diluents,
wherein the pharmaceutical composition is formulated for use in a treatment as set forth in any one of claims 1-14.

16. A pharmaceutical composition, comprising:
(i) a unit dose of an oligomeric compound, wherein the oligomeric compound is an oligomeric compound as defined in claim 1 or claim 2, and
(ii) one or more pharmaceutically acceptable carriers or diluents,
wherein the oligomeric compound is formulated in a sterile liquid; wherein the unit dose of the oligomeric compound is not more than 1 mL, not more than 0.8 mL, not more than 0.5 mL, or not more than 0.25 mL of the sterile liquid; and wherein:
(i) a unit dose comprises not more than 250mg, not more than 100mg, not more than 75mg, not more than 50mg, not more than 40mg, not more than 30mg, not more than 25mg, not more than 20mg, or not more than 15mg of the oligomeric compound, or
(ii) a unit dose comprises:
(A) of from 75mg to 85mg, optionally 80 mg, of the oligomeric compound,
(B) of from 55mg to 65mg, optionally 60 mg, of the oligomeric compound,
(C) of from 45mg to 55mg, optionally 50 mg, of the oligomeric compound,
(D) of from 35mg to 45mg, optionally 40 mg, of the oligomeric compound,
(E) of from 25mg to 35mg, optionally 30 mg, of the oligomeric compound,
(F) of from 15mg to 25mg, optionally 20 mg, of the oligomeric compound, or
(G) of from 5mg to 15mg, optionally 10 mg, of the oligomeric compound, or
(iii) a unit dose comprises not less than 1mg, not less than 2.5mg, or not less than 5mg of the oligomeric compound, or
(iv) a unit dose comprises:
(A) 20 mg of the oligomeric compound,
(B) 40 mg of the oligomeric compound,
(C) 80 mg of the oligomeric compound, or
(D) 120 mg of the oligomeric compound.

17. A sterile sealed container which contains the pharmaceutical composition of claim 15 or claim 16, optionally wherein the container is a vial or a syringe, and optionally further wherein the container is for single use.

18. A packaged pharmaceutical product comprising: (a) multiple unit dosage forms each comprising a sealed sterile container according to claim 17; and (b) printed instructions describing the administration of the unit dosage forms for a treatment as set forth in any of claims 1-15.

## Patentansprüche

1. Oligomere Verbindung zur Verwendung bei der Behandlung oder Vorbeugung einer Krankheit oder eines Leidens bei einem Menschen, wobei die Behandlung Verabreichen von nicht mehr als 300 mg der oligomeren Verbindung an den Menschen während eines Dosierzeitraums von vier Wochen umfasst, wobei die Anionenform der oligomeren Verbindung die folgende chemische Struktur hat:

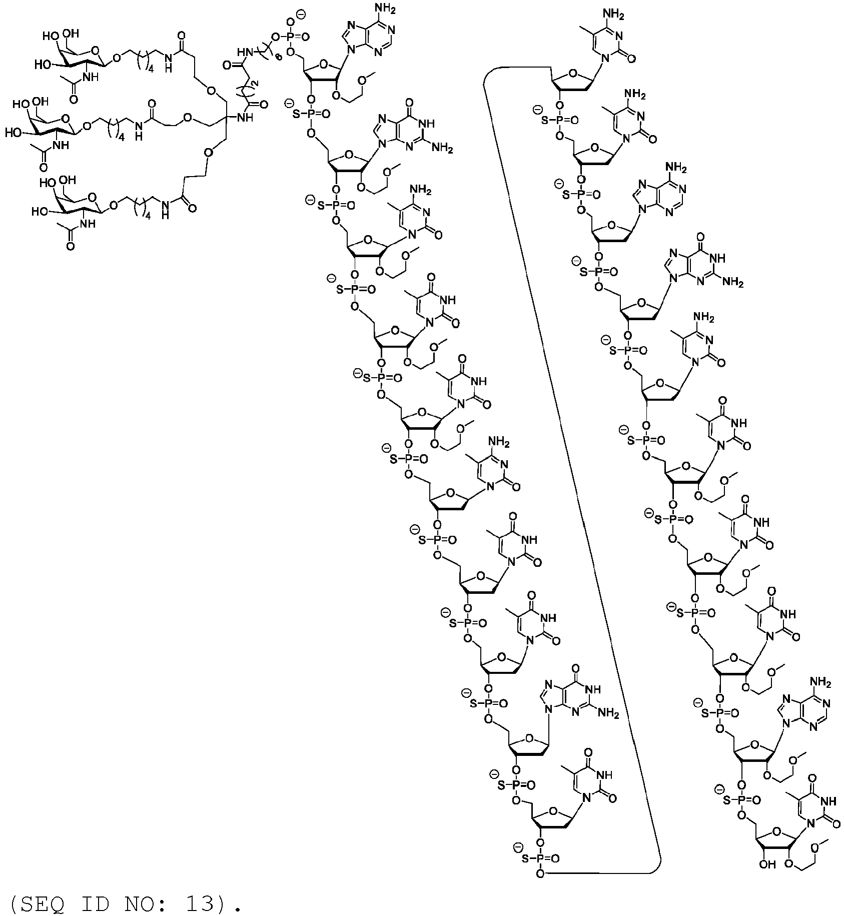

2. Oligomere Verbindung zur Verwendung nach Anspruch 1, wobei es sich bei der oligomeren Verbindung um ein Salz handelt und wobei es sich bei dem Kation des Salzes um Natrium oder Kalium handelt.

3. Oligomere Verbindung zur Verwendung nach einem vorhergehenden Anspruch, wobei die Behandlung Verabreichen von nicht mehr als 250 mg, nicht mehr als 240 mg, nicht mehr als 120 mg, nicht mehr als 100 mg, nicht mehr als 80 mg, nicht mehr als 60 mg, nicht mehr als 40 mg, nicht mehr als 30 mg, nicht mehr als 20 mg, nicht mehr als 10 mg oder nicht mehr als 5 mg der oligomeren Verbindung an den Menschen während des Dosierzeitraums umfasst.

4. Oligomere Verbindung zur Verwendung nach einem vorhergehenden Anspruch, wobei:
(i) die Behandlung Verabreichen einer Einheitsdosis umfasst, die nicht mehr als 250 mg, nicht mehr als 120 mg, nicht mehr als 100 mg, nicht mehr als 80 mg, nicht mehr als 75 mg, nicht mehr als 60 mg, nicht mehr als 50 mg, nicht mehr als 40 mg, nicht mehr als 30 mg, nicht mehr als 25 mg, nicht mehr als 20 mg, nicht mehr als 15 mg, nicht mehr als 10 mg oder nicht mehr als 5 mg der oligomeren Verbindung umfasst, oder
(ii) die Behandlung Verabreichen einer Einheitsdosis
(A) von 115 mg bis 125 mg, gegebenenfalls 120 mg, der oligomeren Verbindung,
(B) von 95 mg bis 105 mg, gegebenenfalls 100 mg, der oligomeren Verbindung,
(C) von 75 mg bis 85 mg, gegebenenfalls 80 mg, der oligomeren Verbindung,
(D) von 55 mg bis 65 mg, gegebenenfalls 60 mg, der oligomeren Verbindung,
(E) von 45 mg bis 55 mg, gegebenenfalls 50 mg, der oligomeren Verbindung,
(F) von 35 mg bis 45 mg, gegebenenfalls 40 mg, der oligomeren Verbindung,
(G) von 25 mg bis 35 mg, gegebenenfalls 30 mg, der oligomeren Verbindung,
(H) von 15 mg bis 25 mg, gegebenenfalls 20 mg, der oligomeren Verbindung,
(I) von 5 mg bis 15 mg, gegebenenfalls 10 mg, der oligomeren Verbindung oder
(J) von 1 mg bis 10 mg, gegebenenfalls 5 mg, der oligomeren Verbindung umfasst oder
(iii) die Behandlung Verabreichen einer Einheitsdosis umfasst, die nicht weniger als 1 mg, nicht weniger als 2,5 mg oder nicht weniger als 5 mg der oligomeren Verbindung umfasst, oder
(iv) die Behandlung Verabreichen einer Einheitsdosis umfasst, die
(A) 20 mg der oligomeren Verbindung,
(B) 40 mg der oligomeren Verbindung,
(C) 80 mg der oligomeren Verbindung oder
(D) 120 mg der oligomeren Verbindung umfasst.

5. Oligomere Verbindung zur Verwendung nach einem vorhergehenden Anspruch, wobei die Behandlung Verabreichen von nicht mehr als 1 Einheitsdosis, nicht mehr als 2 Einheitsdosen, nicht mehr als 3 Einheitsdosen, nicht mehr als 4 Einheitsdosen, nicht mehr als 5 Einheitsdosen oder nicht mehr als 6 Einheitsdosen an den Menschen während des Dosierzeitraums umfasst.

6. Oligomere Verbindung zur Verwendung nach einem der Ansprüche 1-4, wobei der Dosierungsplan der Behandlung Verabreichen einer Einheitsdosis einmal pro Woche, alle zwei Wochen, alle drei Wochen, alle vier Wochen, jeden Monat, alle zwei Monate oder alle drei Monate umfasst.

7. Oligomere Verbindung zur Verwendung nach einem vorhergehenden Anspruch, wobei die oligomere Verbindung durch subkutane Injektion, gegebenenfalls durch subkutane Injektion in das Abdomen, den Oberschenkel oder den Oberarm, verabreicht wird.

8. Oligomere Verbindung zur Verwendung nach einem vorhergehenden Anspruch, wobei die oligomere Verbindung durch Injektion verabreicht wird, gegebenenfalls wobei:
(a) die oligomere Verbindung in einer sterilen Flüssigkeit formuliert ist und gegebenenfalls wobei jede Einheitsdosis der oligomeren Verbindung nicht mehr als 1 ml, nicht mehr als 0,8 ml, nicht mehr als 0,5 ml oder nicht mehr als 0,25 ml der sterilen Flüssigkeit beträgt, gegebenenfalls wobei die sterile Flüssigkeit unter Salzlösung und Wasser ausgewählt ist, gegebenenfalls wobei die sterile Flüssigkeit ferner einen Puffer und/oder Natriumchlorid umfasst, und/oder
(b) die oligomere Verbindung als Natriumsalz formuliert ist.

9. Oligomere Verbindung zur Verwendung nach einem vorhergehenden Anspruch, wobei durch die Behandlung die Nüchterntriglycerid-Plasmakonzentration beim Menschen um wenigstens 30 % verringert wird, wenn die Nüchterntriglycerid-Plasmakonzentration beim Menschen zu Beginn und am Ende des Dosierzeitraums gemessen wird.

10. Oligomere Verbindung zur Verwendung nach einem vorhergehenden Anspruch, wobei die Behandlung die Verabreichung an ein Individuum umfasst, bei dem ein oder mehrere Symptome einer Herz-Kreislauf-Krankheit oder - Störung vorliegen, und wobei durch die Behandlung ein oder mehrere Symptome der Herz-Kreislauf-Krankheit bzw. -Störung verbessert werden.

11. Oligomere Verbindung zur Verwendung nach einem der Ansprüche 1-9, wobei die Behandlung die Verabreichung an ein Individuum umfasst, bei dem eine Herz-Kreislauf-Krankheit, metabolisches Syndrom, Fettleibigkeit und/oder Diabetes vorliegt.

12. Oligomere Verbindung zur Verwendung nach einem der Ansprüche 1-9, wobei die Behandlung die Verabreichung an ein Individuum umfasst, bei dem Hypertriglyceridämie, gemischte Dyslipidämie, Atherosklerose, ein Risiko der Entwicklung von Atherosklerose, koronare Herzkrankheit, ein oder mehrere Risikofaktoren für koronare Herzkrankheit, Diabetes, Lebersteatose und/oder Pankreatitis vorliegt bzw. vorliegen.

13. Oligomere Verbindung zur Verwendung nach Anspruch 12, wobei die Behandlung die Verabreichung an ein Individuum umfasst, bei dem Typ-II-Diabetes und/oder Hypertriglyceridämie vorliegt.

14. Oligomere Verbindung zur Verwendung nach Anspruch 12, wobei die Behandlung die Verabreichung an ein Individuum umfasst, bei dem familiäre Hypertriglyceridämie, nicht familiäre Hypertriglyceridämie, heterozygote familiäre Hypertriglyceridämie und/oder homozygote familiäre Hypertriglyceridämie vorliegt.

15. Pharmazeutische Zusammensetzung, umfassend:
(i) eine oligomere Verbindung, wobei es sich bei der oligomeren Verbindung um eine oligomere Verbindung gemäß Anspruch 1 oder Anspruch 2 handelt, und
(ii) eine oder mehrere pharmazeutisch unbedenkliche Träger oder Verdünnungsmittel,
wobei die pharmazeutische Zusammensetzung zur Verwendung bei einer Behandlung gemäß einem der Ansprüche 1-14 formuliert ist.

16. Pharmazeutische Zusammensetzung, umfassend:
(i) eine Einheitsdosis einer oligomeren Verbindung, wobei es sich bei der oligomeren Verbindung um eine oligomere Verbindung gemäß Anspruch 1 oder Anspruch 2 handelt, und
(ii) eine oder mehrere pharmazeutisch unbedenkliche Träger oder Verdünnungsmittel,
wobei die oligomere Verbindung in einer sterilen Flüssigkeit formuliert ist; wobei die Einheitsdosis der oligomeren Verbindung nicht mehr als 1 ml, nicht mehr als 0,8 ml, nicht mehr als 0,5 ml oder nicht mehr als 0,25 ml der sterilen Flüssigkeit beträgt; und wobei:
(i) eine Einheitsdosis nicht mehr als 250 mg, nicht mehr als 100 mg, nicht mehr als 75 mg, nicht mehr als 50 mg, nicht mehr als 40 mg, nicht mehr als 30 mg, nicht mehr als 25 mg, nicht mehr als 20 mg oder nicht mehr als 15 mg der oligomeren Verbindung umfasst oder
(ii) eine Einheitsdosis Folgendes umfasst:
(A) 75 mg bis 85 mg, gegebenenfalls 80 mg, der oligomeren Verbindung,
(B) 55 mg bis 65 mg, gegebenenfalls 60 mg, der oligomeren Verbindung,
(C) 45 mg bis 55 mg, gegebenenfalls 50 mg, der oligomeren Verbindung,
(D) 35 mg bis 45 mg, gegebenenfalls 40 mg, der oligomeren Verbindung,
(E) 25 mg bis 35 mg, gegebenenfalls 30 mg, der oligomeren Verbindung,
(F) 15 mg bis 25 mg, gegebenenfalls 20 mg, der oligomeren Verbindung oder
(G) 5 mg bis 15 mg, gegebenenfalls 10 mg, der oligomeren Verbindung, oder
(iii) eine Einheitsdosis nicht weniger als 1 mg, nicht weniger als 2,5 mg oder nicht weniger als 5 mg der oligomeren Verbindung umfasst oder
(iv) eine Einheitsdosis
(A) 20 mg der oligomeren Verbindung,
(B) 40 mg der oligomeren Verbindung,
(C) 80 mg der oligomeren Verbindung oder
(D) 120 mg der oligomeren Verbindung umfasst.

17. Steriler verschlossener Behälter, der die pharmazeutische Zusammensetzung gemäß Anspruch 15 oder Anspruch 16 enthält, gegebenenfalls wobei es sich bei dem Behälter um ein Fläschchen oder eine Spritze handelt und gegebenenfalls weiter wobei der Behälter zum Einmalgebrauch bestimmt ist.

18. Abgepacktes pharmazeutisches Produkt, umfassend: (a) mehrere Einheitsdarreichungsformen, die jeweils einen verschlossenen sterilen Behälter nach Anspruch 17 umfassen; und (b) gedruckte Anweisungen, in denen die Verabreichung der Einheitsdarreichungsformen für eine Behandlung gemäß einem der Ansprüche 1-15 beschrieben ist.

## Revendications

1. Composé oligomérique pour une utilisation dans le traitement ou la prévention d'une maladie ou d'une affection chez un humain, le traitement comprenant une administration de pas plus de 300 mg de composé oligomérique chez l'humain pendant une période de dosage de quatre semaines, la forme anionique du composé oligomérique ayant la structure chimique suivante :

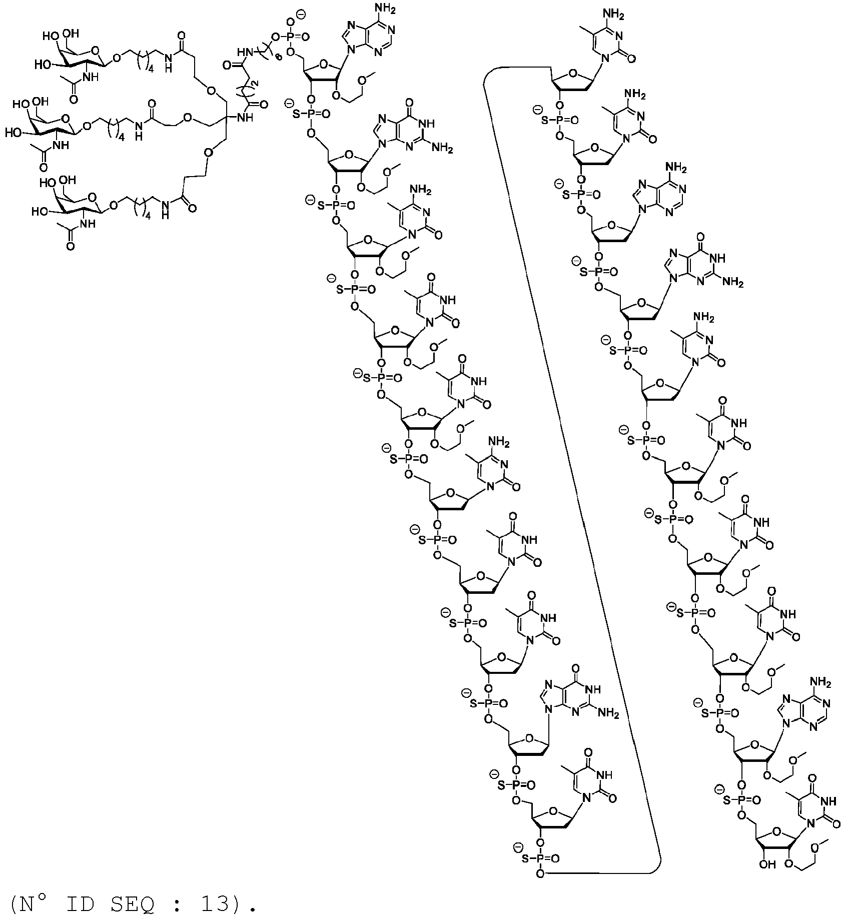

2. Composé oligomérique pour une utilisation selon la revendication 1, le composé oligomérique étant un sel et le cation du sel étant sodium ou potassium.

3. Composé oligomérique pour une utilisation selon une quelconque revendication précédente, le traitement comprenant une administration de pas plus de 250 mg, pas plus de 240 mg, pas plus de 120 mg, pas plus de 100 mg, pas plus de 80 mg, pas plus de 60 mg, pas plus de 40 mg, pas plus de 30 mg, pas plus de 20 mg, pas plus de 10 mg ou pas plus de 5 mg du composé oligomérique chez l'humain pendant la période de dosage.

4. Composé oligomérique pour une utilisation selon une quelconque revendication précédente,
(i) le traitement comprenant une administration d'une dose unitaire ne comprenant pas plus de 250 mg, pas plus de 120 mg, pas plus de 100 mg, pas plus de 80 mg, pas plus de 75 mg, pas plus de 60 mg, pas plus de 50 mg, pas plus de 40 mg, pas plus de 30 mg, pas plus de 25 mg, pas plus de 20 mg, pas plus de 15 mg, pas plus de 10 mg ou pas plus de 5 mg du composé oligomérique, ou
(ii) le traitement comprenant une administration d'une dose unitaire :
(A) allant de 115 mg à 125 mg, éventuellement 120 mg, du composé oligomérique,
(B) allant de 95 mg à 105 mg, éventuellement 100 mg, du composé oligomérique,
(C) allant de 75 mg à 85 mg, éventuellement 80 mg, du composé oligomérique,
(D) allant de 55 mg à 65 mg, éventuellement 60 mg, du composé oligomérique,
(E) allant de 45 mg à 55 mg, éventuellement 50 mg, du composé oligomérique,
(F) allant de 35 mg à 45 mg, éventuellement 40 mg, du composé oligomérique,
(G) allant de 25 mg à 35 mg, éventuellement 30 mg, du composé oligomérique,
(H) allant de 15 mg à 25 mg, éventuellement 20 mg, du composé oligomérique,
(I) allant de 5 mg à 15 mg, éventuellement 10 mg, du composé oligomérique, ou
(J) allant de 1 mg à 10 mg, éventuellement 5 mg, du composé oligomérique, ou
(iii) le traitement comprenant une administration d'une dose unitaire ne comprenant pas moins de 1 mg, pas moins de 2,5 mg ou pas moins de 5 mg du composé oligomérique, ou
(iv) le traitement comprenant une administration d'une dose unitaire comprenant :
(A) 20 mg du composé oligomérique,
(B) 40 mg du composé oligomérique,
(C) 80 mg du composé oligomérique, ou
(D) 120 mg du composé oligomérique.

5. Composé oligomérique pour une utilisation selon une quelconque revendication précédente, le traitement comprenant une administration de pas plus de 1 dose unitaire, pas plus de 2 doses unitaires, pas plus de 3 doses unitaires, pas plus de 4 doses unitaires, pas plus de 5 doses unitaires ou pas plus de 6 doses unitaires chez l'humain pendant la période de dosage.

6. Composé oligomérique pour une utilisation selon l'une quelconque des revendications 1 à 4, le régime de dosage de traitement comprenant une administration d'une dose unitaire une fois par semaine, toutes les deux semaines, toutes les trois semaines, toutes les quatre semaines, tous les mois, tous les deux mois ou tous les trois mois.

7. Composé oligomérique pour une utilisation selon une quelconque revendication précédente, le composé oligomérique étant administré par injection sous-cutanée, éventuellement par injection sous-cutanée dans l'abdomen, la cuisse ou la partie supérieure du bras.

8. Composé oligomérique pour une utilisation selon une quelconque revendication précédente, le composé oligomérique étant administré par injection, éventuellement :
(a) le composé oligomérique étant formulé dans un liquide stérile et éventuellement chaque dose unitaire du composé oligomérique n'étant pas plus de 1 mL, pas plus de 0,8 mL, pas plus de 0,5 mL ou pas plus de 0,25 mL du liquide stérile,
éventuellement le liquide stérile étant choisi parmi : une solution saline et de l'eau, éventuellement le liquide stérile comprenant en outre un tampon et/ou du chlorure de sodium, et/ou
(b) le composé oligomérique étant formulé comme un sel de sodium.

9. Composé oligomérique pour une utilisation selon une quelconque revendication précédente, le traitement réduisant la concentration en triglycéride plasmatique à jeun chez l'humain d'au moins 30 %, lorsque la concentration en triglycéride plasmatique à jeun chez l'humain est mesurée au début et à la fin de la période de dosage.

10. Composé oligomérique pour une utilisation selon une quelconque revendication précédente, le traitement comprenant une administration à un sujet ayant un ou plusieurs symptômes d'une maladie ou d'un trouble cardiovasculaire, et le traitement améliorant un ou plusieurs symptômes de la maladie ou du trouble cardiovasculaire.

11. Composé oligomérique pour une utilisation selon l'une quelconque des revendications 1 à 9, le traitement comprenant une administration à un sujet ayant une maladie cardiovasculaire, un syndrome métabolique, de l'obésité ou un diabète.

12. Composé oligomérique pour une utilisation selon l'une quelconque des revendications 1 à 9, le traitement comprenant une administration à un sujet ayant une hypertriglycéridémie, une dyslipidémie mixte, de l'athérosclérose, un risque de développer l'athérosclérose, une maladie cardiaque coronarienne, un ou plusieurs facteurs de risque pour une maladie cardiaque coronarienne, le diabète, une stéatose hépatique et/ou une pancréatite.

13. Composé oligomérique pour une utilisation selon la revendication 12, le traitement comprenant une administration à un sujet atteint de diabète de type II et/ou d'hypertriglycéridémie.

14. Composé oligomérique pour une utilisation selon la revendication 12, le traitement comprenant une administration à un sujet atteint d'hypertriglycéridémie familiale, d'hypertriglycéridémie non familiale, d'hypertriglycéridémie familiale hétérozygote et/ou d'hypertriglycéridémie familiale homozygote.

15. Composition pharmaceutique, comprenant :
(i) un composé oligomérique, le composé oligomérique étant un composé oligomérique tel que défini dans la revendication 1 ou la revendication 2, et
(ii) un ou plusieurs supports ou diluants pharmaceutiquement acceptables,
la composition pharmaceutique étant formulée pour une utilisation dans un traitement tel qu'indiqué dans l'une quelconque des revendications 1 à 14.

16. Composition pharmaceutique, comprenant :
(i) une dose unitaire d'un composé oligomérique, le composé oligomérique étant un composé oligomérique tel que défini dans la revendication 1 ou la revendication 2, et
(ii) un ou plusieurs supports ou diluants pharmaceutiquement acceptables,
le composé oligomérique étant formulé dans un liquide stérile ; la dose unitaire du composé oligomérique n'étant pas plus de 1 mL, pas plus de 0,8 mL, pas plus de 0,5 mL ou pas plus de 0,25 mL du liquide stérile ; et :
(i) une dose unitaire ne comprenant pas plus de 250 mg, pas plus de 100 mg, pas plus de 75 mg, pas plus de 50 mg, pas plus de 40 mg, pas plus de 30 mg, pas plus de 25 mg, pas plus de 20 mg ou pas plus de 15 mg du composé oligomérique, ou
(ii) une dose unitaire comprenant :
(A) allant de 75 mg à 85 mg, éventuellement 80 mg, du composé oligomérique,
(B) allant de 55 mg à 65 mg, éventuellement 60 mg, du composé oligomérique,
(C) allant de 45 mg à 55 mg, éventuellement 50 mg, du composé oligomérique,
(D) allant de 35 mg à 45 mg, éventuellement 40 mg, du composé oligomérique,
(E) allant de 25 mg à 35mg, éventuellement 30 mg, du composé oligomérique,
(F) allant de 15 mg à 25 mg, éventuellement 20 mg, du composé oligomérique, ou
(G) allant de 5 mg à 15 mg, éventuellement 10 mg, du composé oligomérique, ou
(iii) une dose unitaire ne comprenant pas moins de 1 mg, pas moins de 2,5 mg ou pas moins de 5 mg du composé oligomérique, ou
(iv) une dose unitaire comprenant :
(A) 20 mg du composé oligomérique,
(B) 40 mg du composé oligomérique,
(C) 80 mg du composé oligomérique, ou
(D) 120 mg du composé oligomérique.

17. Récipient scellé stérile qui contient la composition pharmaceutique selon la revendication 15 ou la revendication 16, éventuellement le récipient étant un flacon ou une seringue, et éventuellement en outre le récipient étant pour une utilisation unique.

18. Produit pharmaceutique emballé comprenant : (a) plusieurs formes de dosage unitaires comprenant chacune un récipient stérile scellé selon la revendication 17 ; et (b) des instructions imprimées décrivant l'administration des formes de dosage unitaires pour un traitement tel qu'indiqué dans l'une quelconque des revendications 1 à 15.
